# EUROPEAN PATENT APPLICATION

(11) **EP 4 447 059 A1**
(43) Date of publication of application: **16.10.2024**
(21) Application number: 24169226.8
(22) Date of filing: 09.04.2024
(51) Int. Cl.: G16H 10/60, G16H 40/20, G16H 50/20

(54) **CONSULTATION RECOMMENDATION SYSTEM AND BASE STATION DEVICE**

(30) Priority: 14.04.2023 JP 2023066749
(71) Applicant: Suzuki Motor Corporation, Shizuoka 432-8611 (JP)
(72) Inventor: TAKAGAITO, Fumiya, Shizuoka, 432-8611 (JP); KAMIYA, Naoki, Shizuoka, 432-8611 (JP); NAGAE, Yusuke, Shizuoka, 432-8611 (JP)
(74) Representative: Haseltine Lake Kempner LLP

(57) **Abstract**

A consultation recommendation system 1 includes: a mobility server 40A that stores user data D2; a medical server 40B that stores vehicle data Dl including traveling information; a determination device that determines the presence or absence of an abnormal indication of the user from the vehicle data D1, stores determination result data D4 including a determination result in the medical server 40B, and issues a notification related to a cognitive function to the user based on the determination result data; and user terminals 60, 60A, and 60B, in which when the determination device determines that there is an abnormal indication in the user, the determination device transmits, to a user terminal specified based on the user data corresponding to the determination result, a notification of a consultation recommendation on a disease related to the cognitive function.

## Description

### TECHNICAL FIELD

The present invention relates to a consultation recommendation system and a base station device.

### BACKGROUND ART

For example, Patent Literature 1 discloses a dementia risk determination system in which a situation determination unit determines a driving situation of a vehicle based on road information acquired by a road information acquisition unit and traveling information of the vehicle acquired by a vehicle information acquisition unit, and a violation determination unit determines whether the driving situation corresponds to a predetermined traffic violation that is likely to be performed when a cognitive function deteriorates. In the dementia risk determination system, when the driving situation of the vehicle corresponds to the predetermined traffic violation, a risk determination unit determines whether there is a dementia risk based on information related to a driver of the vehicle and a violation history obtained by a driver information detection unit. When the risk determination unit determines that there is a dementia risk, an output unit outputs the information.

### CITATION LIST

### PATENT LITERATURE

Patent Literature 1: JP2019-124975A

### SUMMARY OF INVENTION

### TECHNICAL PROBLEM

Simple inspection related to the cognitive function performed in the related art is performed based on a temporary state of a person who receives the inspection, and does not confirm a state of the cognitive function from daily behavior such as driving of the vehicle. Therefore, an indication or a symptom of deterioration in cognitive function may not be reflected in an inspection result, and in particular, an abnormality of the cognitive function in an initial stage may not be detected. In addition, even when a subject is notified that an indication or a symptom of the deterioration in cognitive function has been discovered in the simple inspection, the subject may not be able to appropriately determine how to perform the behavior. From such a point of view, it is desired to quickly discover an indication or a symptom of the deterioration in cognitive function from a daily behavior, and to improve a medical examination intention to a medical institution when an indication or a symptom of the deterioration in cognitive function is discovered.

The present invention has been made in view of the above circumstances, and an object thereof is to provide a consultation recommendation system and a base station device capable of appropriately making a consultation recommendation on a disease related to a cognitive function to a user of a vehicle.

### SOLUTION TO PROBLEM

In order to achieve the above object, a consultation recommendation system according to the present invention includes: a mobility server configured to store user data related to a user of a vehicle; a medical server configured to store vehicle data including traveling information of the vehicle; a determination device configured to determine the presence or absence of an abnormal indication of the user from the vehicle data, store determination result data including a determination result in the medical server, and issue a notification related to a cognitive function to the user based on the determination result data; and a user terminal configured to receive the notification from the determination device, in which the mobility server and the medical server cooperate to manage the user data, the vehicle data, and the determination result data in association with each other, and when the determination device determines that there is an abnormal indication in the user, the determination device specifies a user terminal based on the user data corresponding to the determination result, and transmits, to the user terminal, a notification of a consultation recommendation on a disease related to the cognitive function.

In order to achieve the above object, a base station device according to the present invention includes: a mobility server configured to store user data related to a user of a vehicle; a medical server configured to store vehicle data including traveling information of the vehicle; and a determination device configured to determine the presence or absence of an abnormal indication of the user from the vehicle data, store determination result data including a determination result in the medical server, and issue a notification related to a cognitive function to the user based on the determination result data, in which the mobility server and the medical server cooperate to manage the user data, the vehicle data, and the determination result data in association with each other, and when the determination device determines that there is an abnormal indication in the user, the determination device transmits, to a user corresponding to the determination result, a notification of a consultation recommendation on a disease related to the cognitive function.

### ADVANTAGEOUS EFFECTS OF INVENTION

A consultation recommendation system and a base station device according to the present invention have an effect of being capable of appropriately making a consultation recommendation on a disease related to a cognitive function to a user of a vehicle.

### BRIEF DESCRIPTION OF DRAWINGS

[FIG. 1] FIG. 1 is a block diagram showing a schematic configuration of a consultation recommendation system according to a first embodiment.
[FIG. 2] FIG. 2 is a block diagram showing a schematic configuration of the consultation recommendation system according to the first embodiment.
[FIG. 3] FIG. 3 is a schematic diagram illustrating main data treated by the consultation recommendation system according to the first embodiment.
[FIG. 4] FIG. 4 is a block diagram showing a schematic configuration of an in-vehicle device included in the consultation recommendation system according to the first embodiment.
[FIG. 5] FIG. 5 is a block diagram showing a schematic configuration of a data processing device included in the consultation recommendation system according to the first embodiment.
[FIG. 6] FIG. 6 is a block diagram showing a schematic configuration of a mobility server included in the consultation recommendation system according to the first embodiment.
[FIG. 7] FIG. 7 is a block diagram showing a schematic configuration of an analysis device included in the consultation recommendation system according to the first embodiment.
[FIG. 8] FIG. 8 is a block diagram showing a schematic configuration of a medical server included in the consultation recommendation system according to the first embodiment.
[FIG. 9] FIG. 9 is a block diagram showing a schematic configuration of a determination device included in the consultation recommendation system according to the first embodiment.
[FIG. 10] FIG. 10 is a schematic diagram showing an example of a database managed in cooperation between the mobility server and the medical server included in the consultation recommendation system according to the first embodiment.
[FIG. 11] FIG. 11 is a block diagram showing a schematic configuration of a user terminal included in the consultation recommendation system according to the first embodiment.
[FIG. 12] FIG. 12 is a diagram showing an example of a notification of a consultation recommendation displayed on the user terminal included in the consultation recommendation system according to the embodiment.
[FIG. 13] FIG. 13 is a diagram showing an example of the notification of the consultation recommendation displayed on the user terminal included in the consultation recommendation system according to the embodiment.
[FIG. 14] FIG. 14 is a diagram showing an example of the notification of the consultation recommendation displayed on the user terminal included in the consultation recommendation system according to the embodiment.
[FIG. 15] FIG. 15 is a block diagram showing a schematic configuration of a medical institution terminal included in the consultation recommendation system according to the first embodiment.
[FIG. 16] FIG. 16 is a block diagram showing a schematic configuration of a service providing organization terminal included in the consultation recommendation system according to the first embodiment.
[FIG. 17] FIG. 17 is a diagram showing an example of a flow (sequence) of a process performed by each processing unit of the consultation recommendation system according to the first embodiment.
[FIG. 18] FIG. 18 is a diagram showing an example of a flow (sequence) of a process performed by each processing unit of the consultation recommendation system according to the first embodiment.
[FIG. 19] FIG. 19 is a diagram showing an example of a flow (sequence) of a process performed by each processing unit of the consultation recommendation system according to the first embodiment.
[FIG. 20] FIG. 20 is a block diagram showing a schematic configuration of a consultation recommendation system according to a second embodiment.
[FIG. 21] FIG. 21 is a block diagram showing a schematic configuration of an analysis device included in the consultation recommendation system according to the second embodiment.
[FIG. 22] FIG. 22 is a diagram showing an example of a flow (sequence) of a process performed by each processing unit of the consultation recommendation system according to the second embodiment.
[FIG. 23] FIG. 23 is a diagram showing an example of a flow (sequence) of a process performed by each processing unit of the consultation recommendation system according to the second embodiment.

### DESCRIPTION OF EMBODIMENTS

Hereinafter, embodiments according to the present invention will be described in detail with reference to the drawings. The present invention is not limited to these embodiments. In addition, the constituent elements in the following embodiments include components easily replaced by a person skilled in the art or essentially the same components.

### [First Embodiment]

### <Overview of Consultation Recommendation System>

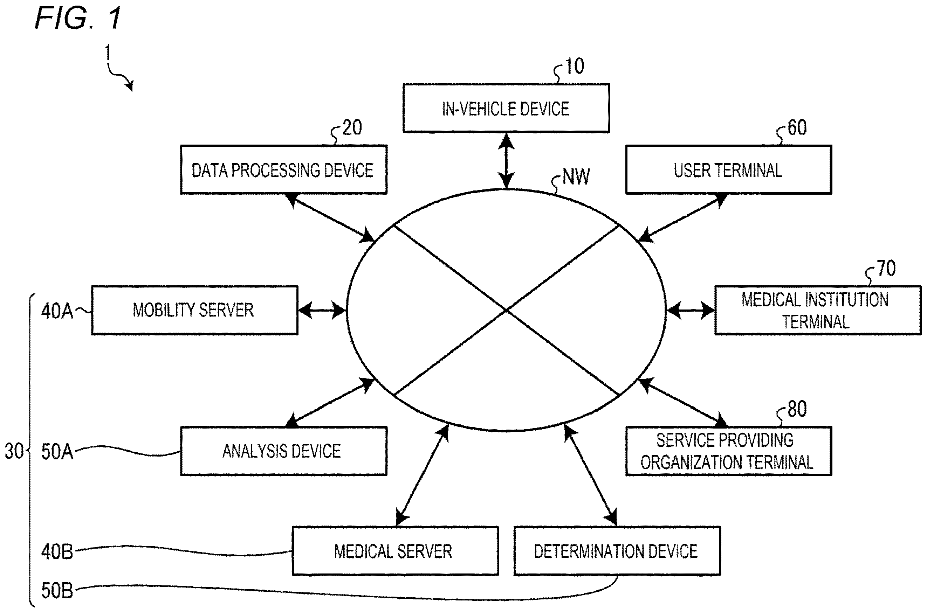

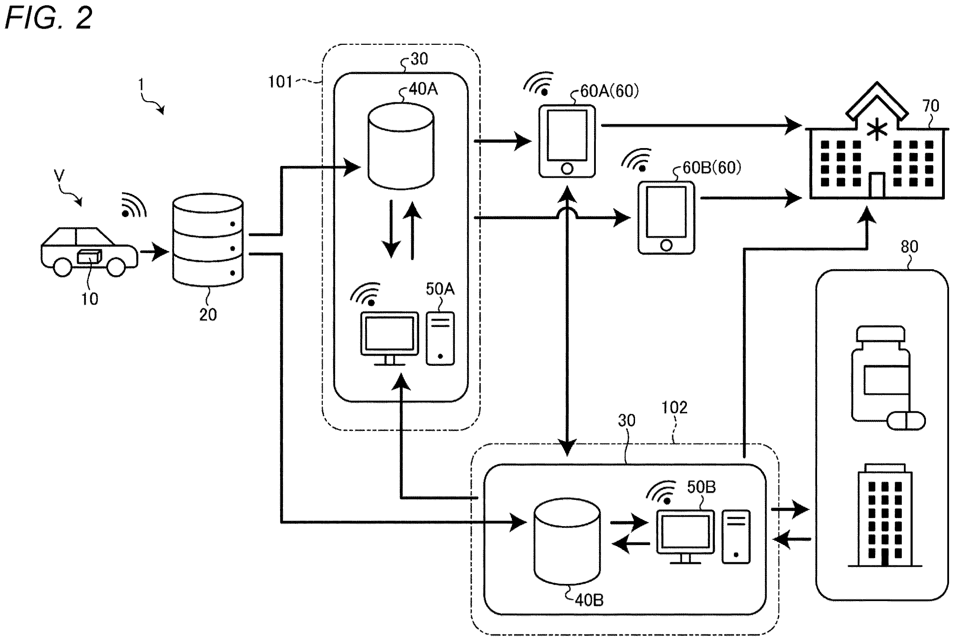

A consultation recommendation system 1 according to a first embodiment shown in FIGS. 1 and 2 includes an in-vehicle device 10, a data processing device 20, a service providing device 30, a user terminal 60, a medical institution terminal 70, and a service providing organization terminal 80, and these devices and terminals are configured to communicate with each other via a network NW. The network NW constitutes a communication network that connects a plurality of devices so as to be able to communicate with each other, and enables data communication by various communication methods that can be applied to the present system regardless of wireless communication or wired communication. The network NW may include, for example, an Internet line network, a mobile phone line network, a mobile communication network, a local area network (LAN), a wide area network (WAN), and a virtual private network (VPN), and various network relay devices such as an antenna, a gateway, a router, and a hub may be interposed. The consultation recommendation system 1 according to the present embodiment constitutes a system in which the in-vehicle device 10, the data processing device 20, the service providing device 30, the user terminal 60, the medical institution terminal 70, and the service providing organization terminal 80 can perform data communication with each other via the network NW to cooperate with each other to manage various kinds of data related to a vehicle V, and the data can be used for various services and analyses.

In such a consultation recommendation system 1, the service providing device 30 according to the present embodiment includes a mobility server 40A, a medical server 40B, an analysis device 50A, and a determination device 50B, and various kinds of data treated by the consultation recommendation system 1 are stored separately in the mobility server 40A and the medical server 40B. Accordingly, independence of the data stored in the mobility server 40A and the medical server 40B can be increased, and confidentiality of information can be protected. The consultation recommendation system 1 according to the present embodiment determines the presence or absence, the content, and the like of an abnormal indication of a user of the vehicle V from a daily driving behavior of the user, and when it is determined that there is an abnormal indication in the user, the consultation recommendation system 1 provides the user with a medical service that issues, to the user, a notification of a consultation recommendation on a disease related to a cognitive function, thereby making an appropriate consultation recommendation for the user.

Here, the abnormal indication of the user who drives the vehicle V refers to an indication of deterioration in cognitive function of the user or an abnormal driving behavior of the user. From researches related to a relation between a cognitive function of a driver and a driving behavior, the present inventor has found that a correlation relation is present between and a cognitive function state of the driver and specific driving behaviors, such as sudden braking, sudden steering, and overlooking of a temporary stop sign. Specifically, the present inventor has found that, compared to a case where a person having a normal cognitive function drives the vehicle V, in a case where a person having an abnormal cognitive function drives the vehicle V, sudden braking, sudden steering, overlooking of a temporary stop sign, or the like tends to increase. The consultation recommendation system 1 according to the present embodiment determines the presence or absence of an indication (an abnormal indication) of deterioration in cognitive function of the user who drives the vehicle V from the vehicle data D1 based on a reference created on the basis of a correlation relation between a driving operation of the vehicle V and a cognitive function state of the user, for example, a reference such as whether sudden braking, sudden steering, overlooking of a temporary stop sign, or the like occurs a predetermined number of times or more in a certain period (for example, in a period in which the vehicle V travels 100 km). The consultation recommendation system 1 according to the present embodiment may determine, as an abnormal indication (an abnormal driving behavior) of the user who drives the vehicle V, that sudden braking, sudden steering, overlooking of a temporary stop sign, or the like occurs the predetermined number of times or more in the period (for example, in the period in which the vehicle V travels 100 km), or that an inter-vehicle distance is shortened compared to past driving, or the like, without determining that an indication of deterioration in cognitive function of the user is present.

In the consultation recommendation system 1 according to the present embodiment, data stored in the mobility server 40A and the medical server 40B is used by the determination device 50B. The determination device 50B determines the presence or absence, the content, and the like of an abnormal indication of each user based on data stored in the mobility server 40A and the medical server 40B. The determination device 50B issues a notification of a consultation recommendation to the user terminal 60 owned by the user who is determined to have an abnormal indication.

The mobility server 40A and the medical server 40B constitute a storage unit in the consultation recommendation system 1 according to the present embodiment. In the storage unit, the mobility server 40A stores user data D2 related to the user of the vehicle V, and the medical server 40B stores vehicle data D1 including traveling information D13 of the vehicle V The determination device 50B includes a determination unit that determines the presence or absence of an abnormal indication of the user from the vehicle data D 1 of the vehicle V, and a notification unit that issues, to the user, a notification of a consultation recommendation on a disease related to the cognitive function when the determination unit determines that there is an abnormal indication in the user. The mobility server 40A and the medical server 40B cooperate with each other to associate and manage the user data D2 and the vehicle data D1.

In the present embodiment, the user terminal 60 typically includes a first user terminal 60A owned by the user himself or herself and a second user terminal 60B owned by a family of the user, a related person, or the like. The determination device 50B transmits, to the user terminal 60, a notification of a consultation recommendation on a disease related to a cognitive function, and thus it is possible to present a behavior recommended for the disease related to the cognitive function, such as proposing the user to have a consultation at a medical institution.

In the present embodiment, the medical institution terminal 70 is typically a terminal device installed at a medical institution such as a university hospital or a general hospital. The medical institution terminal 70 is configured such that, for example, when the user has a consultation at the medical institution, a doctor of the medical institution can acquire information related to an abnormal indication of the user stored in the medical server 40B.

In the present embodiment, the service providing organization terminal 80 is typically a terminal device installed in a service providing organization such as a pharmaceutical manufacturer or an insurance company. The service providing organization terminal 80 is configured such that, for example, the service providing organization can acquire information related to an abnormal indication of the user stored in the medical server 40B. For example, when the service providing organization acquires the information related to an abnormal indication of the user, the service providing organization terminal 80 is configured to issue, to the user terminal 60, a notification of service information corresponding to the information acquired by the service providing organization via the service providing device 30.

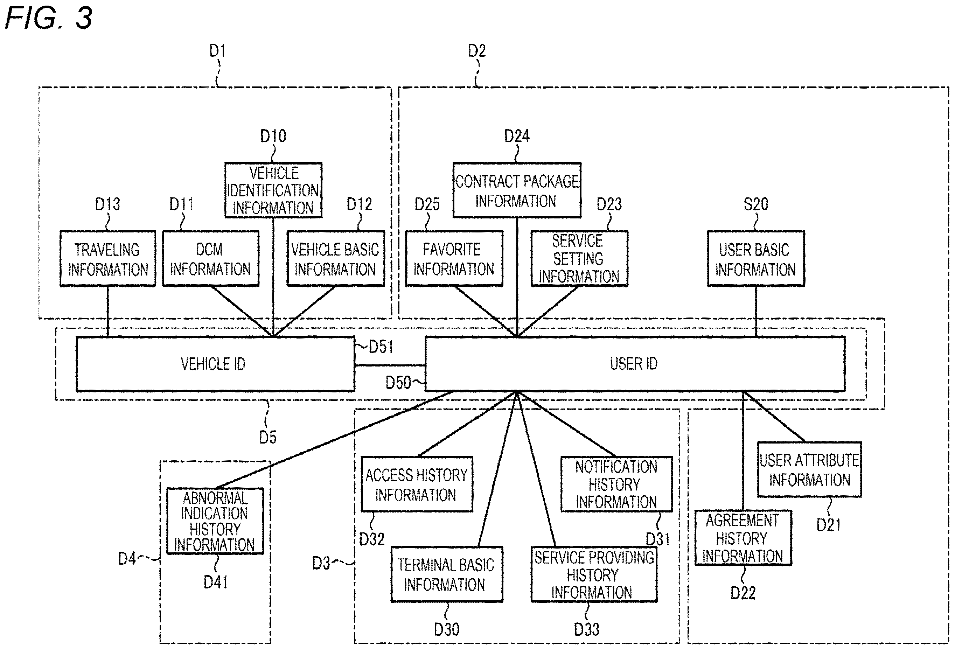

Hereinafter, configurations of the consultation recommendation system 1 will be described in detail with reference to the drawings. In the following, first, an outline of main data treated by the consultation recommendation system 1 will be described with reference to FIG. 3, and thereafter, each configuration of the consultation recommendation system 1 will be described in detail with reference to other drawings and the like.

In the consultation recommendation system 1, the vehicle V to be treated may be any driving system such as a gasoline vehicle, a diesel vehicle, a hybrid electric vehicle (HEV), a plug-in hybrid electric vehicle (PHEV), or an electric vehicle (EV). A driving method of the vehicle V may be any of manual driving, semi-automatic driving, fully automatic driving, and the like. In addition to the own vehicle of the user who can enjoy a service provided by the consultation recommendation system 1, the vehicle V may be any one of a truck, a bus, a taxi, a special vehicle, and the like that can be identified as being used by the user.

### <Main Data Treated by Consultation Recommendation System>

The main data treated by the consultation recommendation system 1 includes, for example, the vehicle data D1, the user data D2, terminal data D3, determination result data D4, and ID data D5 as illustrated in FIG. 3. The vehicle data D1, the user data D2, the terminal data D3, and the determination result data D4 are managed in association with the ID data D5 and the like in the consultation recommendation system 1.

### <Vehicle Data>

The vehicle data D1 is data related to the vehicle V Typically, the vehicle data D1 is data collected by the in-vehicle device 10 mounted on the vehicle V and transmitted to a service providing device 30 side. Here, the vehicle data D1 includes, for example, data related to vehicle identification information D10, DCM information D11, vehicle basic information D12, and traveling information D13.

The vehicle identification information D10 is unique information assigned to each vehicle V to identify the vehicle V, and is, for example, information such as a vehicle identification number (VIN). In addition, the vehicle identification information D10 may be information such as a vehicle registration number (so-called car number) or a frame number of a vehicle. The DCM information D11 is information such as a type or an international mobile equipment identifier (IMEI) of a data communication module (DCM) mounted on the vehicle V The vehicle basic information D12 is information such as a vehicle name, a vehicle type, a model, and a grade of the vehicle V The traveling information D13 is information related to a traveling situation of the vehicle V In the present embodiment, the traveling information D13 includes driving information, vehicle state information, vehicle position information, device operation information, and image information.

The driving information is basic information related to driving of the vehicle V, and is, for example, information such as a vehicle speed, acceleration/deceleration, fuel consumption, an accumulated traveling distance, and a battery remaining amount. The driving information may include information related to the weather during traveling or information related to sound in the vehicle during traveling. The vehicle state information is detailed information related to a state of the vehicle V, and is, for example, information such as a steering wheel steering angle, an engine coolant temperature, an accelerator opening degree (an accelerator operation amount), a brake opening degree (an accelerator operation amount), an engine rotation speed, an engine torque, a motor torque, and a motor rotation speed. The vehicle position information is information such as position information (GNSS information, GPS information) of the vehicle V The device operation information is information related to an operation state of a device mounted on the vehicle V, and is, for example, information such as a door lock opening and closing state, a hazard lamp lighting state, a direction indicator operation state, a vehicle warning lamp lighting state, and presence or absence of an emergency notification. The image information is, for example, information such as a moving image inside and outside the vehicle captured in the vehicle V In addition, for example, information such as an acquisition date and time of information is added to the vehicle data D1.

In addition to the above, the vehicle data D1 may include, for example, data related to biological information or the like of an occupant of the vehicle V The biological information of the occupant may include, in addition to various kinds of physiological information generated by a living body (the occupant), various kinds of information derived from the physiological information, and is, for example, vital sign information such as a heart rate, a respiratory rate, a pulse rate, a blood pressure, and a body temperature, biological identification information such as a fingerprint, a voiceprint, and an iris, and information such as a blood alcohol concentration and an arousal level.

### <User Data>

The user data D2 is data related to the user of the vehicle V Here, the user in the consultation recommendation system 1 is the driver of the vehicle V, and is a person who determines the presence or absence of an abnormal indication by the consultation recommendation system 1 and receives a consultation recommendation related to the cognitive function when it is determined that an abnormal indication is present. A service utilizer who enjoys a service provided by the consultation recommendation system or a contractor who has made a contract for receiving the service is also included in users who use the consultation recommendation system 1. That is, the user data D2 can also be referred to as utilizer data of a service provided by the consultation recommendation system 1, a contractor data related to the service, or customer data of a business operator who develops the service. Typically, the user data D2 is data registered in the mobility server 40A by the in-vehicle device 10, the user terminal 60, or the like, or data automatically generated on a mobility server 40A side based on the data registered in the mobility server 40A. Here, the user data D2 includes, for example, data related to user basic information D20, user attribute information D21, agreement history information D22, service setting information D23, contract package information D24, favorite information D25, and the like.

The user basic information D20 is information related to a contractor (a customer) registered by the user himself or herself, and is, for example, information such as a name, a zip code, an address, a mail address, a telephone number, and a work contact address of the user. The user attribute information D21 is information related to an attribute of the user registered by the user himself or herself or automatically generated on a mobility server 40A side, and is, for example, information such as age, gender, occupation, and nationality. The agreement history information D22 is information related to various agreement histories automatically generated on the mobility server 40A side, and is, for example, information related to presence or absence of agreement with respect to a privacy policy or a service policy. Here, the agreement history information D22 includes information related to the presence or absence of agreement of the user to provide a determination result (the determination result data D4) of the presence or absence of an abnormal indication by the vehicle data D1 or the determination device 50B to the medical institution or the service providing organization. The service setting information D23 is setting information necessary for enjoying a service registered by the user himself or herself, and is, for example, information such as a language setting and a notification setting in various services. Here, the service setting information D23 includes terminal registration information of the user terminal 60. The terminal registration information of the user terminal 60 is information related to the user terminal 60 registered by the user himself or herself or automatically generated on the mobility server 40A side, and is information capable of specifying the user terminal 60 of a notification destination when the determination device 50B issues, to the user, a notification of a consultation recommendation on a disease related to a cognitive function. The contract package information D24 is information related to a contract automatically generated on the mobility server 40A side, and is, for example, information such as a contract status (valid/expiration/cancellation), a contract start date/expiration date/cancellation date, and a contract plan. The favorite information D25 is information related to a preference of the user registered by the user himself or herself, and is, for example, information related to a home hospital, a medical institution that the user wants to have a consultation, an insurance company that is under contract, and a favorite agency (dealer). In addition, for example, information such as an acquisition date and time of the information is added to the user data D2.

### <Terminal Data>

The terminal data D3 is data related to a notification history to the user terminal 60, the medical institution terminal 70, and the service providing organization terminal 80, and an access history from the medical institution terminal 70 and the service providing organization terminal 80. Typically, the terminal data D3 is data collected in association with a notification to the user terminal 60, the medical institution terminal 70, and the service providing organization terminal 80 or an access from the service providing organization terminal 80, in the service providing device 30. The terminal data D3 includes, for example, data related to terminal basic information D30, notification history information D31, access history information D32, service providing history information D33, and the like.

The terminal basic information D30 is basic information capable of specifying the user terminal 60, the medical institution terminal 70, and the service providing organization terminal 80, and includes information such as a type of each terminal, an owner of each terminal, and an organization owned by each terminal. The notification history information D31 is information related to a notification from the service providing device 30 to the user terminal 60, the medical institution terminal 70, and the service providing organization terminal 80, and is, for example, information related to a transmission history of a notification of a consultation recommendation to the user terminal 60, a history of a notification of the vehicle data D1 and the determination result data D4 to the medical institution terminal 70, a history of a notification of the vehicle data D1 and the determination result data D4 to the service providing organization terminal 80, and the like. The service providing history information D33 is information such as a history of a notification related to a service of a service providing organization received from the service providing organization terminal 80. In addition, for example, information such as an acquisition date and time of each information is added to the terminal data D3.

### <Determination Result Data>

The determination result data D4 is information including a determination result related to an abnormal indication of the user generated by the determination device 50B. Here, the determination device 50B determines the presence or absence, the content, and the like of an abnormal indication of the user based on a reference (a first reference) created on the basis of a correlation relation between the driving operation of the vehicle V and the cognitive function state of the user or a reference (a second reference) whether the driving operation corresponds to a predetermined driving operation which is an abnormal driving behavior, and generates a determination result as determination result data D4. The determination result data D4 includes abnormal indication history information D41. The abnormal indication history information D41 is information such as the presence or absence of an abnormal indication of the user, the content of the abnormal indication of the user, and the date and time when it is determined that there is an abnormal indication in the user.

### <ID Data>

The ID data D5 is an identifier that can manage the vehicle data D1, the user data D2, the terminal data D3, and the determination result data D4 described above in association with each other. The ID data D5 includes, for example, data related to a user ID D50, a vehicle ID D51, and the like. The user ID D50 is a unique identifier assigned to each user to identify the user. The user data D2, the terminal data D3, and the determination result data D4 described above are managed in association with the user ID D50. On the other hand, the vehicle ID D51 is a unique identifier assigned to each vehicle V in order to identify the vehicle V Here, in order to identify and manage the vehicle V, the vehicle ID D51 is set separately from the vehicle identification information D10 described above. The vehicle data D1 is managed in association with the vehicle ID D51.

The user ID D50 and the vehicle ID D51 are associated with each other in association with the vehicle V used by the user. For example, when one user owns a plurality of vehicles V, a relation between the user ID D50 and the vehicle ID D51 is that a plurality of vehicle IDs D51 are associated with one user ID D50. As a result, the vehicle data D1, the user data D2, the terminal data D3, and the determination result data D4 can be associated with each other for each user corresponding to each user ID D50 based on the user ID D50 or the like. That is, the vehicle data D1 related to the vehicle V used by a specific user, and the user data D2 related to the specific user, the terminal data D3, and the determination result data D4 can be managed as a set of data sets associated with each other via the user ID D50 and the vehicle IDs D51. As described above, when the plurality of vehicle IDs D51 are associated with one user ID D50, a plurality of pieces of vehicle data D1 are respectively associated with the user data D2 of the corresponding user, the terminal data D3, and the determination result data D4 via the user ID D50 and the vehicle IDs D51. On the other hand, when one vehicle V is used by a plurality of users, a relation between the user ID D50 and the vehicle ID D51 is that a plurality of user IDs D50 are associated with one vehicle ID D51.

An overview of the main data treated by the consultation recommendation system 1 has been described above. Although the consultation recommendation system 1 also treats data other than the data described above, the data will be appropriately described later.

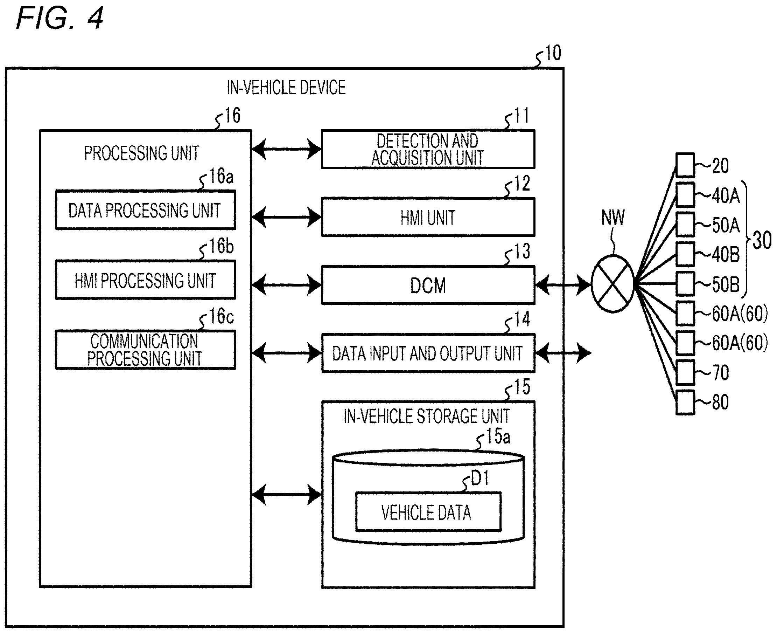

Next, configurations of the consultation recommendation system 1 will be described with reference to FIGS. 4 to 14. In the following description, various kinds of data may be appropriately referred to in FIG. 3.

### <Basic Configuration of In-vehicle Device>

The in-vehicle device 10 shown in FIG. 4 is a device that is mounted on the vehicle V and that collects and stores the vehicle data D1 related to the vehicle V in association with the vehicle identification information D10 of the vehicle V The in-vehicle device 10 is capable of communicating with an external device of the vehicle V For example, the in-vehicle device 10 may include a function of a so-called drive recorder or the like that is mounted on the vehicle V and records various kinds of information, and may be configured to be installed in the vehicle V later.

Specifically, as shown in FIG. 4, the in-vehicle device 10 includes a detection and acquisition unit 11, a human machine interface (HMI) unit 12, a DCM 13, a data input and output unit 14, an in-vehicle storage unit 15, and a processing unit 16. The detection and acquisition unit 11, the HMI unit 12, the DCM 13, the data input and output unit 14, the in-vehicle storage unit 15, and the processing unit 16 are communicably connected to each other.

The detection and acquisition unit 11 is a device that detects and acquires various kinds of information included in the vehicle data D1 related to the vehicle V The detection and acquisition unit 11 detects and acquires the vehicle identification information D10, the DCM information D11, the vehicle basic information D12, the traveling information D13, and the like as the vehicle data D1. The detection and acquisition unit 11 includes, for example, various sensors provided in units of the vehicle V, a detector, a GPS receiver, a microphone, and an imaging device. The detection and acquisition unit 11 may include, for example, a device that acquires various kinds of information from an electronic control unit (ECU) that integrally controls the entire vehicle V When the biological information or the like of the occupant of the vehicle V is included in the vehicle data D1, the detection and acquisition unit 11 may include a device that acquires various kinds of information in cooperation with a monitoring device that monitors the occupant or a wearable terminal worn by the occupant.

The HMI unit 12 is a device that performs various inputs and outputs in the in-vehicle device 10. The HMI unit 12 may include, for example, a keyboard, a switch, an input button, a mouse pointer, a trackball, a joystick, a touch pad, a microphone, and a voice input device, as an operation input device that performs various inputs to the in-vehicle device 10. The HMI unit 12 is implemented as an information output device that performs various outputs from the in-vehicle device 10. Here, the information output device is a touch panel device or the like capable of outputting information to a user and receiving an operation input from the user. The HMI unit 12 may include, for example, a speaker and a display, as an information output device that performs various outputs from the in-vehicle device 10.

The DCM 13 is a communication module capable of communicating with an external device of the vehicle V The DCM 13 is communicably connected to the network NW by wireless communication, and communicates with the external device of the vehicle V (typically, the data processing device 20) via the network NW.

The data input and output unit 14 is a data input and output device that inputs and outputs data (information) to and from the in-vehicle device 10. The data input and output unit 14 is implemented by a recording medium interface or the like, and reads and writes various kinds of data from and to a recording medium such as a flexible disk (FD), a solid state drive (SSD), a hard disk drive (HDD), a magneto-optical disk, a USB memory, an SD card memory, an Flash memory, a CD-ROM, or a DVD.

The in-vehicle storage unit 15 is a storage circuit that stores various kinds of data. The in-vehicle storage unit 15 may be, for example, a storage device having a relatively large capacity such as an HDD, an SSD, or an optical disk, or a semiconductor memory capable of rewriting data such as a RAM, a flash memory, or a non volatile static random access memory (NVSRAM). The in-vehicle storage unit 15 stores, for example, programs that enable units of the in-vehicle device 10 to implement various functions. The in-vehicle storage unit 15 stores various kinds of data necessary for various kinds of processes in the processing unit 16, and the processing unit 16 or the like reads the various kinds of data as necessary.

The in-vehicle storage unit 15 according to the present embodiment includes a vehicle data storage unit 15a in terms of functional concept.

The vehicle data storage unit 15a is a storage area for storing the vehicle data D1 in the in-vehicle storage unit 15. The vehicle data storage unit 15a stores, as the vehicle data D1, data related to the vehicle identification information D10, the DCM information D11, the vehicle basic information D12, the traveling information D13, and the like detected and acquired by the detection and acquisition unit 11.

The processing unit 16 is a processing circuit that integrally controls the in-vehicle device 10 and implements various processing functions of the in-vehicle device 10. The processing unit 16 is implemented by, for example, a processor, a RAM, and a ROM. The processor means a circuit such as a central processing unit (CPU), a micro processing unit (MPU), a field programmable gate array (FPGA), or an application specific integrated circuit (ASIC). The processing unit 16 implements processing functions, for example, by executing the programs read from the in-vehicle storage unit 15. For example, the processing unit 16 is capable of executing a process of detecting and acquiring the vehicle data D1 by the detection and acquisition unit 11 and storing the vehicle data D1 in the vehicle data storage unit 15a, a process of operating the HMI unit 12, a process of performing data communication via the DCM 13, and the like.

The processing unit 16 according to the present embodiment includes a data processing unit 16a, an HMI processing unit 16b, and a communication processing unit 16c in terms of functional concept in order to implement the various processing functions described above. The processing unit 16 implements processing functions of the data processing unit 16a, the HMI processing unit 16b, and the communication processing unit 16c, for example, by reading and executing the programs (not shown) stored in the in-vehicle storage unit 15.

The data processing unit 16a is a unit having a function capable of executing a process related to various kinds of data in the in-vehicle device 10. The data processing unit 16a is capable of executing a process of detecting and acquiring information constituting the vehicle data D1 by the detection and acquisition unit 11. The data processing unit 16a is capable of executing a process of storing, in the vehicle data storage unit 15a, information detected or acquired by the detection and acquisition unit 11 as the vehicle data D1. The data processing unit 16a is capable of executing a process of reading the vehicle data D1 from the vehicle data storage unit 15a in response to a request. The data processing unit 16a is capable of executing a process of inputting or outputting data to or from the in-vehicle device 10 via the data input and output unit 14. The data processing unit 16a is also capable of executing a process of storing data input via the data input and output unit 14 in the in-vehicle storage unit 15 and a process of reading, from the in-vehicle storage unit 15, data output via the data input and output unit 14. The data processing unit 16a is also capable of executing a process of storing data registered in accordance with an operation on an operation input device constituting the HMI unit 12 in the in-vehicle storage unit 15 and a process of reading the data from the in-vehicle storage unit 15.

For example, the data processing unit 16a may control the detection and acquisition unit 11 to detect or acquire information constituting the vehicle data D1 at a predetermined sampling cycle, or may detect or acquire the information at an appropriate timing. In addition, the data processing unit 16a may acquire a part of the vehicle data D1, such as the vehicle identification information D10, the DCM information D11, and the vehicle basic information D12, registered in accordance with an operation on the operation input device constituting the HMI unit 12. When storing the vehicle data D1 in the vehicle data storage unit 15a, the data processing unit 16a stores the vehicle identification information D10 of the vehicle V on which the in-vehicle device 10 is mounted and the vehicle data D1 in association with each other in the vehicle data storage unit 15a. At this time, for example, the data processing unit 16a can store the vehicle data D1 in the vehicle data storage unit 15a along a time series together with information on a date and time when the vehicle data D1 is collected or the like.

The HMI processing unit 16b is a unit having a function capable of controlling the HMI unit 12 and executing a process of operating the HMI unit 12. The HMI processing unit 16b is capable of executing a process of controlling the operation input device constituting the HMI unit 12 and inputting an operation via the operation input device. The HMI processing unit 16b is capable of executing a process of controlling an information output device constituting the HMI unit 12 and outputting information via the information output device.

The communication processing unit 16c is a unit having a function capable of executing a process of controlling the DCM 13 and performing data communication. The communication processing unit 16c is capable of executing a process of transmitting and receiving data to and from other devices such as the data processing device 20 via the DCM 13. More specifically, the communication processing unit 16c is capable of executing a process of transmitting the vehicle data D1 to the data processing device 20 via the DCM 13 in a state where the vehicle identification information D10 and the corresponding vehicle data D1 are associated with each other. The communication processing unit 16c is capable of executing a process of receiving various commands, requests, and various kinds of information from the data processing device 20 via the DCM 13. The communication processing unit 16c is capable of executing a process of transmitting various commands, requests, and various kinds of information to the data processing device 20 via the DCM 13.

For example, the communication processing unit 16c can transmit the vehicle data D1 stored in the vehicle data storage unit 15a to the data processing device 20 at an appropriate timing. In this case, for example, the communication processing unit 16c may sequentially transmit the vehicle data D1 to the data processing device 20 in real time in accordance with a sampling cycle of the detection power acquisition unit 11, or may collectively transmit the vehicle data D1 of a plurality of sampling cycles to the data processing device 20 at a cycle different from the sampling cycle of the detection power acquisition unit 11. For example, the communication processing unit 16c may transmit the vehicle data D1 to the data processing device 20 at any timing in accordance with an operation on the operation input device constituting the HMI unit 12, or may transmit the vehicle data D1 to the data processing device 20 at a timing when a request such as a transmission command from an external device (the data processing device 20 or the like) is received. In this case, for example, the in-vehicle device 10 can be expected to have an effect of reducing congestion due to an increase in a communication traffic amount. For example, the communication processing unit 16c can change a transmission cycle of the vehicle data D1 according to the state of the vehicle V In this case, for example, when the vehicle V is stopped, a significant change in the vehicle speed, the position information, or the like is not expected, and thus the communication processing unit 16c may transmit the vehicle data D1 at a transmission cycle that is relatively longer than a transmission cycle when the vehicle V is traveling. In this case, for example, the in-vehicle device 10 can transmit data at an appropriate timing in accordance with the state of the vehicle V

### <Basic Configuration of Data Processing Device>

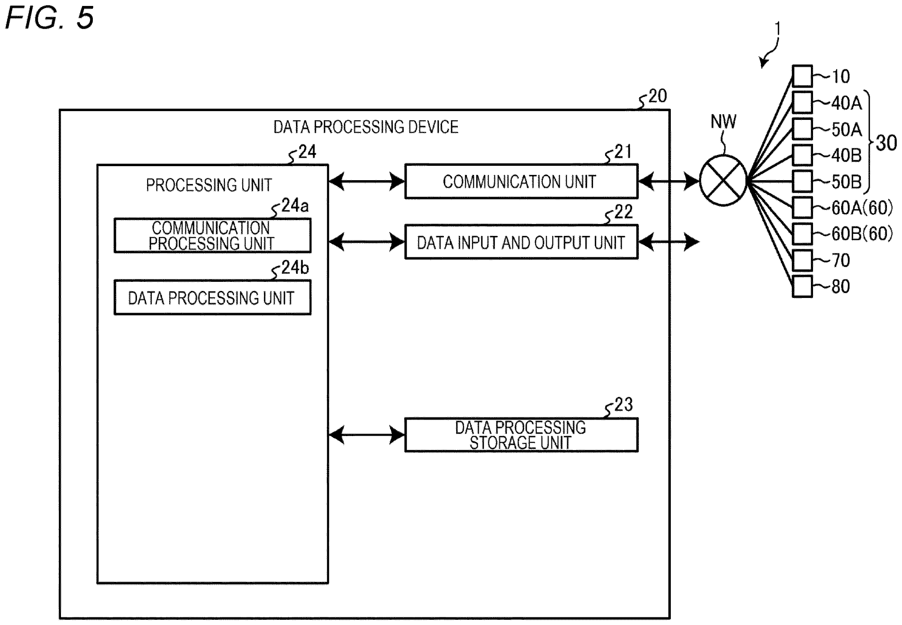

The data processing device 20 shown in FIG. 5 is a network relay device that relays data communication between the in-vehicle device 10 and the service providing device 30 (for example, the mobility server 40A and the medical server 40B). In the present embodiment, the data processing device 20 relays data to be transmitted from the in-vehicle device 10 to the service providing device 30. The service providing device 30 stores the data received from the data processing device 20 in the mobility server 40A and the medical server 40B. The data processing device 20 may be implemented by, for example, various computer devices. The data processing device 20 can also be implemented by, for example, installing programs for realizing various processes in a known computer device.

Specifically, as shown in FIG. 5, the data processing device 20 includes a communication unit 21, a data input and output unit 22, a data processing storage unit 23, and a processing unit 24. The communication unit 21, the data input and output unit 22, the data processing storage unit 23, and the processing unit 24 are communicably connected to each other.

The communication unit 21 is a communication module capable of communicating with devices other than the data processing device 20. The communication unit 21 is communicably connected to the network NW regardless of wireless communication or wired communication, and communicates with other devices (for example, the in-vehicle device 10 and the service providing device 30) via the network NW.

The data input and output unit 22 is a data input and output device that inputs and outputs data (information) to and from the data processing device 20. The data input and output unit 22 has substantially the same configuration as the data input and output unit 14 described above, and is implemented by a recording medium interface or the like.

The data processing storage unit 23 is a storage circuit that stores various kinds of data. The data processing storage unit 23 has substantially the same configuration as the in-vehicle storage unit 15 described above, and is implemented by, for example, an HDD, an SSD, an optical disk, and a semiconductor memory. The data processing storage unit 23 stores, for example, programs that enable units of the data processing device 20 to implement various functions. The data processing storage unit 23 stores various kinds of data necessary for various kinds of processes in the processing unit 24, and the processing unit 24 or the like reads the various kinds of data as necessary

The processing unit 24 is a processing circuit that integrally controls the data processing device 20 and implements various processing functions of the data processing device 20. The processing unit 24 has substantially the same configuration as the processing unit 16 described above, and is implemented by, for example, a processor, a RAM, and a ROM. The processing unit 24 implements processing functions, for example, by reading and executing the programs stored in the data processing storage unit 23. For example, the processing unit 24 is capable of executing a process of performing data communication via the communication unit 21, a process of relaying various kinds of data, a process of transmitting data received from the in-vehicle device 10 to the mobility server 40A and the medical server 40B and storing the data, and the like.

The processing unit 24 according to the present embodiment includes a communication processing unit 24a and a data processing unit 24b in terms of functional concept in order to implement the various processing functions described above. The processing unit 24 implements processing functions of the data processing unit 24b and the communication processing unit 24a, for example, by reading and executing the programs stored in the data processing storage unit 23.

The communication processing unit 24a is a unit having a function capable of executing a process of controlling the communication unit 21 and performing data communication. The communication processing unit 24a is capable of executing a process of transmitting and receiving data to and from other devices such as the in-vehicle device 10 and the service providing device 30 via the communication unit 21. More specifically, for example, the communication processing unit 24a is capable of executing a process of receiving the vehicle data D1 from the in-vehicle device 10 via the communication unit 21. The communication processing unit 24a is capable of executing a process of transmitting the vehicle data D1 to the service providing device 30 (for example, the mobility server 40A and the medical server 40B) via the communication unit 21. The communication processing unit 24a is capable of executing a process of receiving various commands and requests from the in-vehicle device 10, the service providing device 30, and the like via the communication unit 21. The communication processing unit 24a is capable of executing a process of transmitting various commands, requests, and various kinds of information to the in-vehicle device 10 and the service providing device 30 (for example, the mobility server 40A and the medical server 40B) via the communication unit 21.

For example, when relaying data between the devices, the communication processing unit 24a executes processes such as a process of blocking unauthorized communication, a process of ensuring communication with a high security level such as encrypted communication, a routing process of distributing a transmission destination of data, and a protocol conversion process of converting a communication protocol between different networks.

The data processing unit 24b is a unit having a function capable of executing a process related to various kinds of data in the data processing device 20. The data processing unit 24b is capable of executing a process of storing data received via the communication unit 21 in the data processing storage unit 23. The data processing unit 24b is capable of executing a process of reading data transmitted via the communication unit 21 from the data processing storage unit 23. The data processing unit 24b is capable of executing a process of inputting and outputting data to and from the data processing device 20 via the data input and output unit 22. The data processing unit 24b is also capable of executing a process of storing data input via the data input and output unit 22 in the data processing storage unit 23 and a process of reading data output via the data input and output unit 22 from the data processing storage unit 23.

The communication processing unit 24a transmits the vehicle data D1 read from the data processing storage unit 23 by the data processing unit 24b to the mobility server 40A and the medical server 40B via the communication unit 21.

### <Basic Configuration of Service Providing Device>

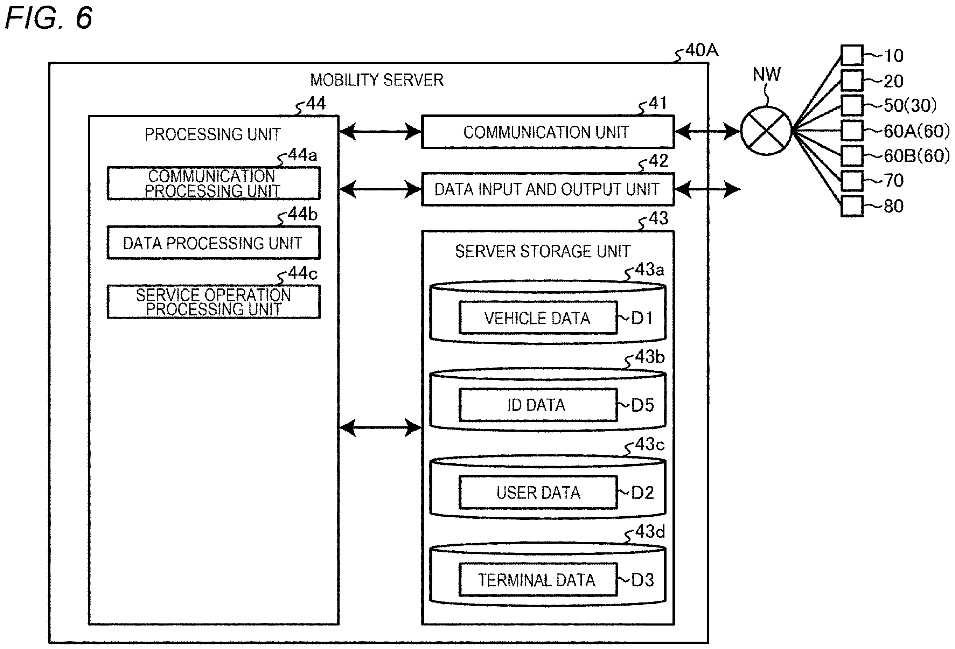

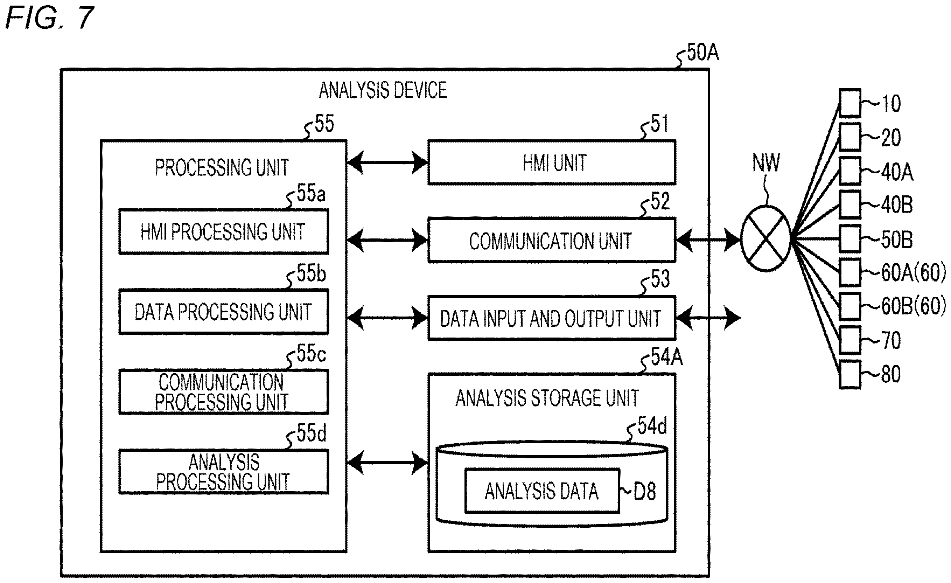

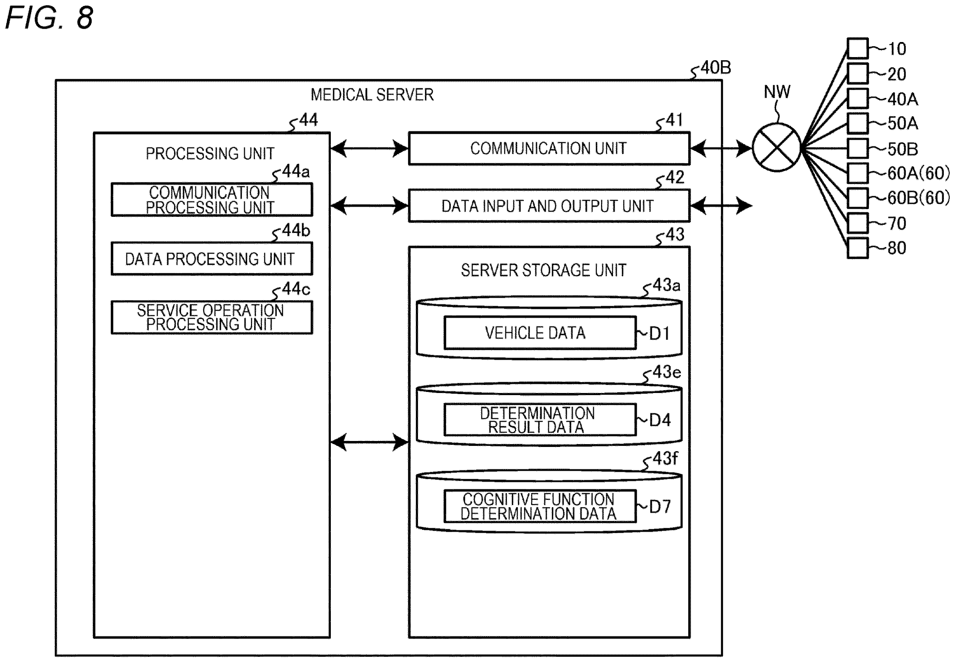

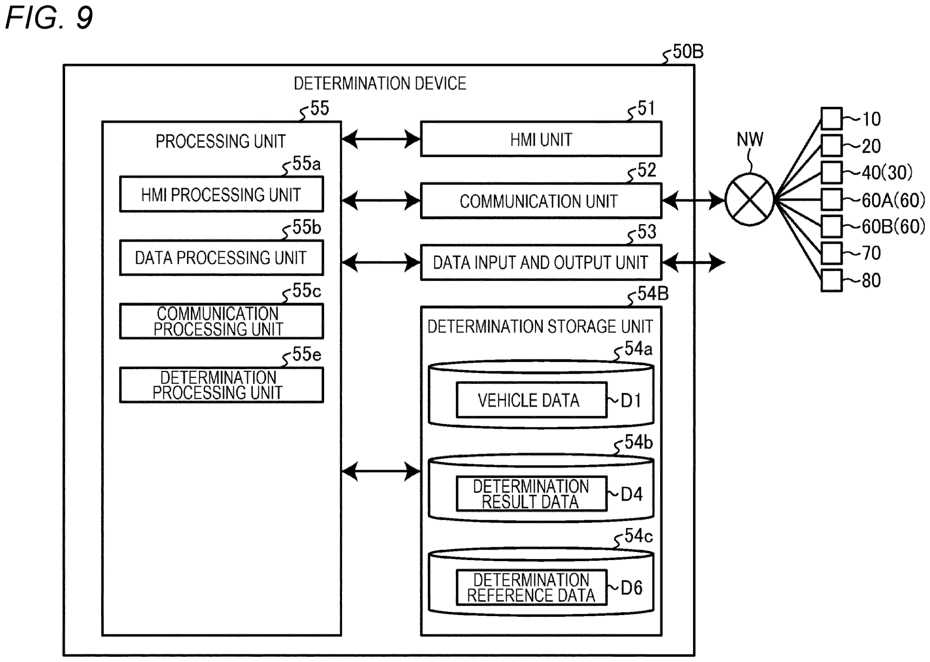

The service providing device 30 is capable of collectively managing various kinds of data treated by the consultation recommendation system 1, determining the presence or absence, the content, and the like of an abnormal indication of the user based on the vehicle data D1, and issuing, to the user, a notification of a consultation recommendation on a disease related to a cognitive function when it is determined that there is an abnormal indication in the user. The service providing device 30 includes the mobility server 40A shown in FIG. 6, the analysis device 50A shown in FIG. 7, the medical server 40B shown in FIG. 8, and the determination device 50B shown in FIG. 9. Typically, the service providing device 30 constitutes a base station device provided at a base station that relays communication between the vehicle V and the user terminal 60. In the present embodiment, the base station device (the service providing device 30) includes a first base station device disposed at a mobility base station 101 and including the mobility server 40A and the analysis device 50A, and a second base station device disposed at a medical base station 102 and including the medical server 40B and the determination device 50B. The mobility base station 101 is, for example, a base station managed by a business operator or an analysis business operator of the vehicle V The medical base station 102 is, for example, a base station managed by a business operator that develops various medical services. The analysis device 50A and the determination device 50B do not need to be disposed at the mobility base station 101 and the medical base station 102, and may be disposed at different locations from these base stations. That is, at least the mobility server 40A and the medical server 40B may constitute the base station device. In this case, the mobility server 40A constitutes the first base station device, and the medical server 40B constitutes the second base station device. In this case, for example, the analysis device 50A may be configured to access the first base station device from a remote location different from the location of the mobility base station 101, and the determination device 50B may be configured to access the second base station device from a remote location different from the location of the medical base station 102.

In the service providing device 30 according to the present embodiment, the determination device 50B is configured to communicate with the user terminal 60 via the mobility server 40A and the medical server 40B. The determination device 50B transmits the notification of the consultation recommendation on the disease related to the cognitive function to the user terminal 60 via the mobility server 40A and the medical server 40B.

The mobility server 40A, the analysis device 50A, the medical server 40B, and the determination device 50B may be implemented by, for example, various computer devices. The mobility server 40A, the analysis device 50A, the medical server 40B, and the determination device 50B can also be implemented by, for example, installing programs for implementing various processes in a known computer device. In the present embodiment, the mobility server 40A and the medical server 40B are configured separately and independently as physically different servers. The mobility server 40A and the medical server 40B manage various data in cooperation with each other. The mobility server 40A and the medical server 40B constitute a storage unit of the consultation recommendation system 1 in cooperation with each other. The mobility server 40A and the medical server 40B may be implemented by, for example, various computer devices. The mobility server 40A and the medical server 40B can also be implemented by, for example, installing programs for implementing various processes in a known computer device. Here, the mobility server 40A and the medical server 40B may not be limited to being separately and independently configured as different physical servers, or may be separately and independently configured as so-called virtual servers on the same physical server or a plurality of physical servers according to various virtualization techniques. That is, the mobility server 40A and the medical server 40B are configured to hold data independently of each other. As described above, the mobility server 40A and the medical server 40B may be physically and structurally independent of each other, or may be configured such that, for example, each component such as a processor is physically common and storage areas are independent of each other.

The mobility server 40A and medical server 40B included in the service providing device 30 are typically configured as so-called relational servers that store various kinds of data in a table format such as CSV or Excel, but are not limited thereto. For example, the mobility server 40A and the medical server 40B may be configured as so-called NoSQL servers that store various kinds of data in a format such as XML or JSON. In this case, for example, the mobility server 40A and the medical server 40B can be configured to easily search for a large amount of data at the same time.

### <Basic Configuration of Mobility Server>

The mobility server 40A is configured to cooperate with the medical server 40B, and is a server that mainly manages data used for a service provided to the user of the vehicle V The mobility server 40A and the medical server 40B are connected servers that cooperate with each other to deploy connected services. In the consultation recommendation system 1 according to the present embodiment, a service is performed in which a determination related to an abnormal indication of a user who drives the vehicle V is performed using data (the vehicle data D1) stored in the medical server 40B, and a notification of a consultation recommendation is transmitted to the user terminal 60 when it is determined that there is an abnormal indication in the user. In the consultation recommendation system 1 according to the present embodiment, not only services for notifying the consultation recommendation, but also services such as contract management, parking position search, vehicle remote control, emergency notification support, trouble support, security notification, and driving history evaluation can be provided to the user by using data stored in the mobility server 40A.

Specifically, as shown in FIG. 6, the mobility server 40A includes a communication unit 41, a data input and output unit 42, a server storage unit 43, and a processing unit 44. The communication unit 41, the data input and output unit 42, the server storage unit 43, and the processing unit 44 are communicably connected to each other.

The communication unit 41 is a communication module capable of communicating with devices other than the mobility server 40A. The communication unit 41 has substantially the same configuration as the communication unit 21 described above, is communicably connected to the network NW regardless of wireless communication or wired communication, and communicates with other devices (for example, the data processing device 20 and the user terminal 60) via the network NW.

The data input and output unit 42 is a data input and output device that inputs and outputs data (information) to and from the mobility server 40A. The data input and output unit 42 has substantially the same configuration as the data input and output unit 14 described above, and is implemented by a recording medium interface or the like.

The server storage unit 43 is a storage circuit that stores various kinds of data. The server storage unit 43 has substantially the same configuration as the in-vehicle storage unit 15 described above, and is implemented by, for example, an HDD, an SSD, an optical disk, and a semiconductor memory. The server storage unit 43 stores, for example, programs that enable units of the mobility server 40A to implement various functions (not shown). The server storage unit 43 stores various kinds of data necessary for various kinds of processes in the processing unit 44, and the processing unit 44 or the like reads the various kinds of data as necessary.

The server storage unit 43 according to the present embodiment includes a vehicle data storage unit 43a, an ID data storage unit 43b, a user data storage unit 43c, and a terminal data storage unit 43d in terms of functional concept.

The vehicle data storage unit 43a is a storage circuit that stores various kinds of data. The vehicle data storage unit 43a has substantially the same configuration as the in-vehicle storage unit 15 described above, and is implemented by, for example, an HDD, an SSD, an optical disk, and a semiconductor memory. The vehicle data storage unit 43a is a storage area for storing the vehicle data D1 in the server storage unit 43. The vehicle data storage unit 43a stores the vehicle data D1 transmitted from the in-vehicle device 10. The vehicle data storage unit 43a stores the vehicle data D1 including data related to the vehicle identification information D10, the DCM information D11, the vehicle basic information D12, the traveling information D13, and the like.

The ID data storage unit 43b is a storage area for storing the ID data D5 in the server storage unit 43. The ID data storage unit 43b stores, for example, the ID data D5 including data related to the user ID D50, the vehicle ID D51, and the like registered via the user terminal 60 or automatically generated by the processing unit 44 of the mobility server 40A.

The user data storage unit 43c is a storage area for storing the user data D2 in the server storage unit 43. The user data storage unit 43c stores, for example, the user data D2 including data related to the user basic information D20, the user attribute information D21, the agreement history information D22, the service setting information D23, the contract package information D24, the favorite information D25, and the like registered via the in-vehicle device 10, the user terminal 60, or the like, or automatically generated by the processing unit 44 of the mobility server 40A.

The terminal data storage unit 43d is a storage area for storing the terminal data D3 in the server storage unit 43. The terminal data storage unit 43d stores the terminal data D3 including data related to the terminal basic information D30, the notification history information D31, the access history information D32, the service providing history information D33, and the like of the user terminal 60.

The processing unit 44 is a processing circuit that integrally controls the mobility server 40A and implements various processing functions of the mobility server 40A. The processing unit 44 has substantially the same configuration as the processing unit 16 described above, and is implemented by, for example, a processor, a RAM, and a ROM. The processing unit 44 implements processing functions, for example, by reading and executing the program stored in the server storage unit 43. For example, the processing unit 44 is capable of executing a process of performing data communication via the communication unit 41, a process of specifying the user data D2 corresponding to the determination result data D4 stored in the medical server 40B, a process of calculating various kinds of data, a process of providing various services to the user, and the like.

The processing unit 44 according to the present embodiment includes a communication processing unit 44a, a data processing unit 44b, and a service operation processing unit 44c in terms of functional concept in order to implement the various processing functions described above. The processing unit 44 implements processing functions of the communication processing unit 44a, the data processing unit 44b, and the service operation processing unit 44c, for example, by reading and executing the program stored in the server storage unit 43.

The communication processing unit 44a is a unit having a function capable of executing a process of controlling the communication unit 41 and performing data communication. The communication processing unit 44a is capable of executing a process of transmitting and receiving data to and from other devices such as the data processing device 20 and the user terminal 60 via the communication unit 41.

For example, the communication processing unit 44a is capable of executing a process of receiving the ID data D5 from the user terminal 60 or the like via the communication unit 41. The communication processing unit 44a is capable of executing a process of receiving the vehicle data D1 transmitted from the data processing device 20 to the mobility server 40A via the communication unit 41. The communication processing unit 44a is capable of executing a process of receiving the user data D2 from the in-vehicle device 10 and the user terminal 60 via the communication unit 41. The communication processing unit 44a is capable of executing a process of transmitting a notification of a consultation recommendation to the user terminal 60 via the communication unit 41 in response to a request from the determination device 50B. The communication processing unit 44a is capable of executing a process of receiving various commands and requests from the data processing device 20, the determination device 50B, the user terminal 60, or the like via the communication unit 41.

The data processing unit 44b is a unit having a function capable of executing a process related to various kinds of data in the mobility server 40A. The data processing unit 44b is capable of executing a process of storing data received via the communication unit 41 in the server storage unit 43. The data processing unit 44b is capable of executing a process of reading data transmitted via the communication unit 41 from the server storage unit 43. The data processing unit 44b is also capable of executing a process of storing data input via the data input and output unit 42 in the server storage unit 43 and a process of reading data output via the data input and output unit 42 from the server storage unit 43.

More specifically, for example, the data processing unit 44b is capable of executing a process of storing the vehicle data D1 transmitted from the in-vehicle device 10 via the data processing device 20 in the vehicle data storage unit 43a. The data processing unit 44b is capable of executing a process of reading the vehicle data D1 from the vehicle data storage unit 43a in response to a request.

The data processing unit 44b is capable of executing a process of storing, in the ID data storage unit 43b, data related to the user ID D50, the vehicle ID D51, or the like registered via the user terminal 60 or the like as the ID data D5. The data processing unit 44b may execute a process of automatically generating data related to the user ID D50, the vehicle ID D51, and the like based on information registered via the user terminal 60 or the like, and storing the generated data as the ID data D5 in the ID data storage unit 43b. The data processing unit 44b is capable of executing a process of reading the ID data D5 from the ID data storage unit 43b in response to a request.

The data processing unit 44b is capable of executing a process of storing, in the user data storage unit 43c, data related to the user basic information D20, the service setting information D23, the favorite information D25, and the like registered via the user terminal 60 or the like as the user data D2. The data processing unit 44b is capable of executing a process of automatically generating data related to a part of the user data D2 stored in the mobility server 40A, that is, the user attribute information D21, the agreement history information D22, the contract package information D24, and the like based on information registered via the user terminal 60 or the like, and storing the generated data as the user data D2 in the user data storage unit 43c. For example, the data processing unit 44b may store data registered via the in-vehicle device 10 as a part of the user data D2 in the user data storage unit 43c and form the user data D2. The data processing unit 44b is capable of executing a process of reading the user data D2 from the user data storage unit 43c in response to a request.

The data processing unit 44b is capable of executing a process of storing, in the terminal data storage unit 43d, data related to the terminal basic information D30, the notification history information D31, the access history information D32, the service providing history information D33, and the like transmitted from the user terminal 60 or the like as the terminal data D3. The data processing unit 44b is capable of executing a process of receiving the terminal data D3 from the user terminal 60 via the communication unit 41. The data processing unit 44b is capable of executing a process of storing, in the terminal data storage unit 43d, data related to information transmitted from the determination device 50B to the user terminal 60 via the communication unit 41 as the terminal data D3. The data processing unit 44b is capable of executing a process of acquiring, from the determination device 50B via the communication unit 41, data related to information transmitted from the determination device 50B to the medical institution terminal 70 or the service providing organization terminal 80 and storing the acquired data as the terminal data D3 in the terminal data storage unit 43d. The data processing unit 44b is capable of executing a process of acquiring, from the determination device 50B via the communication unit 41, data related to access information from the service providing organization terminal 80 to the determination device 50B, generating the acquired data as the terminal data D3, and storing the generated data in the terminal data storage unit 43d. The data processing unit 44b is capable of executing a process of acquiring, from the determination device 50B via the communication unit 41, data related to information transmitted from the service providing organization terminal 80 to the determination device 50B, generating the acquired data as the terminal data D3, and storing the generated data in the terminal data storage unit 43d. The data processing unit 44b is capable of executing a process of reading the terminal data D3 from the terminal data storage unit 43d in response to the request.

As described above, when respectively storing the vehicle data D1, the user data D2, and the terminal data D3 in the vehicle data storage unit 43a, the user data storage unit 43c, and the terminal data storage unit 43d, the data processing unit 44b is capable of executing a process of associating the vehicle data D1, the user data D2, and the terminal data D3 with each other. For example, the data processing unit 44b associates, via the vehicle identification information D10 and the user ID D50 and the vehicle ID D51 constituting the ID data D5, the vehicle data D1, the user data D2, and the terminal data D3 stored in the vehicle data storage unit 43a, the user data storage unit 43c, and the terminal data storage unit 43d with each other to form a data set.

### <Basic Configuration of Analysis Device>

The analysis device 50A shown in FIG. 7 relays data transmission and reception between the mobility server 40A and the medical server 40B. The analysis device 50A is capable of performing various analyses such as quality investigation, accident analysis, failure analysis, feedback to development, mobility planning, and utilization in new business based on the data stored in the mobility server 40A. In this case, the analysis device 50A is used, for example, by a developer or an analysis business operator of the vehicle V

Specifically, the analysis device 50A includes an HMI unit 51, a communication unit 52, a data input and output unit 53, an analysis storage unit 54A, and a processing unit 55. The HMI unit 51, the communication unit 52, the data input and output unit 53, the analysis storage unit 54A, and the processing unit 55 are communicably connected to each other.

The HMI unit 51 is a device that performs various inputs and outputs in the determination device 50B. Similarly to the HMI unit 12 described above, the HMI unit 51 includes an operation input device that performs various inputs to the determination device 50B and an information output device that performs various outputs from the analysis device 50A.

The communication unit 52 is a communication module capable of communicating with devices other than the analysis device 50A. The communication unit 52 has substantially the same configuration as the above-described communication unit 21, is communicably connected to the network NW regardless of wireless communication or wired communication, and communicates with other devices (for example, the data processing device 20, the mobility server 40A, the medical server 40B, the user terminal 60, the medical institution terminal 70, and the service providing organization terminal 80) via the network NW.

The data input and output unit 53 is a data input and output device that inputs and outputs data (information) to and from the analysis device 50A. The data input and output unit 53 has substantially the same configuration as the data input and output unit 14 described above, and is implemented by a recording medium interface or the like.

The analysis storage unit 54A is a storage circuit that stores various kinds of data. The analysis storage unit 54Ahas substantially the same configuration as the in-vehicle storage unit 15 described above, and is implemented by, for example, an HDD, an SSD, an optical disk, and a semiconductor memory. The analysis storage unit 54A stores, for example, a program that enables units of the analysis device 50A to implement various functions. The analysis storage unit 54A stores various kinds of data necessary for various kinds of processes in the processing unit 55, and the processing unit 55 or the like reads the various kinds of data as necessary.

The processing unit 55 is a processing circuit that integrally controls the analysis device 50A and implements various processing functions of the analysis device 50A. The processing unit 55 has substantially the same configuration as the processing unit 16 described above, and is implemented by, for example, a processor, a RAM, and a ROM. The processing unit 55 implements processing functions, for example, by reading and executing the program stored in the analysis storage unit 54A. For example, the processing unit 55 is capable of executing a process of operating the HMI unit 51, a process of performing data communication via the communication unit 52, and the like.

The processing unit 55 according to the present embodiment includes an HMI processing unit 55a, a data processing unit 55b, a communication processing unit 55c, and an analysis processing unit 55d in order to implement the various processing functions of the analysis device 50A. The processing unit 55 implements processing functions of the HMI processing unit 55a, the data processing unit 55b, the communication processing unit 55c, and the analysis processing unit 55d by reading and executing the program stored in the analysis storage unit 54A, for example.

Similarly to the HMI processing unit 16b described above, the HMI processing unit 55a is a unit having a function capable of executing a process of controlling the HMI unit 51 and operating the HMI unit 51. The HMI processing unit 55a is capable of executing a process of controlling an operation input device constituting the HMI unit 51 and inputting an operation via the operation input device. The HMI processing unit 55a is capable of executing a process of controlling an information output device constituting the HMI unit 51 and outputting information via the information output device.

Similarly to the data processing unit 44b described above, the data processing unit 55b is a unit having a function capable of executing a process related to various kinds of data in the analysis device 50A. The data processing unit 55b is capable of executing a process of storing data received via the communication unit 52 in the analysis storage unit 54A. The data processing unit 55b is capable of executing a process of reading data transmitted via the communication unit 52 from the analysis storage unit 54A. The data processing unit 55b is also capable of executing a process of storing data input via the data input and output unit 53 in the analysis storage unit 54A and a process of reading data output via the data input and output unit 53 from the analysis storage unit 54A. The data processing unit 55b is also capable of executing a process of storing registered data in the analysis storage unit 54A in accordance with an operation on the operation input device constituting the HMI unit 51, and a process of reading the data from the analysis storage unit 54A.

Similarly to the communication processing unit 44a described above, the communication processing unit 55c is a unit having a function capable of executing a process of controlling the communication unit 52 and performing data communication. The communication processing unit 55c is capable of executing a process of transmitting and receiving data to and from other devices such as the data processing device 20, the mobility server 40A, the medical server 40B, the user terminal 60, the medical institution terminal 70, and the service providing organization terminal 80 via the communication unit 52.

The analysis storage unit 54A of the analysis device 50A includes an analysis data storage unit 54d in terms of functional concept. The analysis data storage unit 54d is a storage area for storing analysis data D8 including analysis results obtained when the analysis device 50A performs various analyses based on data stored in the mobility server 40A.

The communication processing unit 55c of the analysis device 50A is capable of executing a process of transmitting various commands and requests to the medical server 40B via the communication unit 52. The communication processing unit 55c of the analysis device 50A is capable of executing a process of receiving data related to various kinds of information from the medical server 40B via the communication unit 52.

The analysis processing unit 55d executes a process of acquiring the vehicle data D1 and the like from the mobility server 40A in accordance with an operation on the operation input device constituting the HMI unit 51. In response to receiving data necessary for executing the requested analysis in the analysis device 50A from the vehicle data D1 and the like stored in the mobility server 40A, the analysis processing unit 55d analyzes the data based on the data. The data processing unit 55b of the analysis device 50A is capable of executing a process of storing the analysis result obtained by the analysis processing unit 55d as the analysis data D8 in the analysis storage unit 54A. The HMI processing unit 55a of the analysis device 50A is capable of executing a process of controlling the information output device constituting the HMI unit 51 and outputting the analysis data D8 such as an analysis result obtained by the analysis processing unit 55d via the information output device.

### <Basic Configuration of Medical Server>

As described above, the medical server 40B shown in FIG. 8 is a server that mutually cooperates with the mobility server 40A and manages data mainly used for the service provided to the user of the vehicle V

A basic configuration of the medical server 40B is substantially the same as that of the mobility server 40A. The medical server 40B includes the communication unit 41, the data input and output unit 42, the server storage unit 43, and the processing unit 44. The communication unit 41, the data input and output unit 42, the server storage unit 43, and the processing unit 44 are communicably connected to each other.

The communication unit 41 of the medical server 40B is a communication module capable of communicating with devices other than the medical server 40B. The communication unit 41 has substantially the same configuration as the communication unit 21 described above, is communicably connected to the network NW regardless of wireless communication or wired communication, and communicates with other devices (for example, the data processing device 20 and the user terminal 60) via the network NW.

The data input and output unit 42 of the medical server 40B is a data input and output device that inputs and outputs data (information) to and from the medical server 40B. The data input and output unit 42 has substantially the same configuration as the data input and output unit 14 described above, and is implemented by a recording medium interface or the like.

The server storage unit 43 of the medical server 40B has substantially the same configuration as the server storage unit 43 of the mobility server 40A. Similarly to the server storage unit 43 of the mobility server 40A, the server storage unit 43 of the medical server 40B stores various kinds of data necessary for various kinds of processes in the processing unit 44, and the processing unit 44 or the like reads the various kinds of data as necessary.

Here, the server storage unit 43 of the medical server 40B includes a vehicle data storage unit 43a, a determination result data storage unit 43e, and a cognitive function determination data storage unit 43f in terms of functional concept.

The vehicle data storage unit 43a is a storage circuit that stores various kinds of data. The vehicle data storage unit 43a has substantially the same configuration as the vehicle data storage unit 43a of the mobility server 40A described above. The vehicle data storage unit 43a stores the vehicle data D1 transmitted from the in-vehicle device 10. The vehicle data storage unit 43a stores, for example, the vehicle data D1 including data related to the vehicle identification information D10, the DCM information D11, the vehicle basic information D12, the traveling information D13, and the like.

The determination result data storage unit 43e is a storage area for storing the determination result data D4 in the server storage unit 43. The determination result data storage unit 43e stores the determination result data D4 including data related to the abnormal indication history information D41 and the like.

The cognitive function determination data storage unit 43f is a storage area for storing cognitive function determination data D7 which is information related to a general cognitive function test. The user can take the cognitive function test in the user terminal 60 by communicating with the medical server 40B through the user terminal 60 and accessing the cognitive function determination data D7. The cognitive function test simply tests whether the cognitive function is deteriorated, for example, a mini-mental state examination inspection (MMSE inspection) or a response to a check list indicating whether the symptom corresponds to a specific symptom of dementia.

The processing unit 44 is a processing circuit that integrally controls the medical server 40B and implements various processing functions of the medical server 40B. The processing unit 44 has substantially the same configuration as the processing unit 44 of the mobility server 40A. For example, the processing unit 44 is capable of executing a process of performing data communication via the communication unit 41, a process of calculating various kinds of data, a process of providing various services to the user, and the like.

The processing unit 44 according to the present embodiment includes a communication processing unit 44a, a data processing unit 44b, and a service operation processing unit 44c in terms of functional concept in order to implement the various processing functions described above. The processing unit 44 implements processing functions of the communication processing unit 44a, the data processing unit 44b, and the service operation processing unit 44c, for example, by reading and executing the programs stored in the server storage unit 43.

The communication processing unit 44a is a unit having a function capable of executing a process of controlling the communication unit 41 and performing data communication. The communication processing unit 44a is capable of executing a process of transmitting and receiving data to and from other devices such as the data processing device 20 and the user terminal 60 via the communication unit 41.

For example, the communication processing unit 44a can receive an access to the user terminal 60 or the like via the communication unit 41. The communication processing unit 44a is capable of executing a process of receiving the vehicle data D1 transmitted from the data processing device 20 to the medical server 40B via the communication unit 41. The communication processing unit 44a is capable of executing a process of receiving various commands and requests from the data processing device 20, the determination device 50B, the user terminal 60, or the like via the communication unit 41.

Similarly to the mobility server 40A, the data processing unit 44b is a unit having a function capable of executing a process related to various kinds of data in the medical server 40B. The data processing unit 44b is capable of executing a process of storing data received via the communication unit 41 in the server storage unit 43. The data processing unit 44b is capable of executing a process of reading data transmitted via the communication unit 41 from the server storage unit 43. The data processing unit 44b is also capable of executing a process of storing data input via the data input and output unit 42 in the server storage unit 43 and a process of reading data output via the data input and output unit 42 from the server storage unit 43.

More specifically, for example, the data processing unit 44b of the medical server 40B is capable of executing a process of storing the vehicle data D 1 transmitted from the in-vehicle device 10 via the data processing device 20 in the vehicle data storage unit 43a. The data processing unit 44b of the medical server 40B is capable of executing a process of storing the determination result data D4 transmitted from the determination device 50B in the determination result data storage unit 43e. The data processing unit 44b is capable of executing a process of reading the vehicle data D1 from the vehicle data storage unit 43a in response to a request. The data processing unit 44b is capable of executing a process of reading the determination result data D4 from the determination result data storage unit 43e in response to a request.

As described above, when respectively storing the vehicle data D1 and the determination result data D4 in the vehicle data storage unit 43a and the determination result data storage unit 43e, the data processing unit 44b of the medical server 40B is capable of executing a process of associating the vehicle data D1 and the determination result data D4 with each other. For example, the data processing unit 44b associates the vehicle data D1 stored in the vehicle data storage unit 43a and the determination result data D4 stored in the determination result data storage unit 43e with each other via the vehicle identification information D10 to form a data set. That is, the server storage unit 43 of the medical server 40B stores the vehicle data D1 stored in the vehicle data storage unit 43 a and the determination result data D4 stored in the determination result data storage unit 43e by the data processing unit 44b associating the above data with each other based on the vehicle identification information D10, and stores the above data in a database as a data set.

### <Basic Configuration of Determination Device>

The determination device 50B shown in FIG. 9 acquires the vehicle data D1 from the medical server 40B, performs determination related to the presence or absence or the like of an abnormal indication of the user of the vehicle V based on the acquired vehicle data D1, and generates a determination result as determination result data. The determination device 50B transmits the determination result data to the medical server 40B and stores the determination result data. In response to determining that there is an abnormal indication in the user in the determination related to an abnormal indication of the user, the determination device 50B issues, to the user terminal 60, a notification of a consultation recommendation on a disease related to a cognitive function. The determination device 50B is a device that is managed and used by a business operator that mainly provides various services related to medical care, or the like.

Specifically, the determination device 50B includes an HMI unit 51, a communication unit 52, a data input and output unit 53, a determination storage unit 54B, and a processing unit 55. The HMI unit 51, the communication unit 52, the data input and output unit 53, the determination storage unit 54B, and the processing unit 55 are communicably connected to each other.

The HMI unit 51 is a device that performs various inputs and outputs in the determination device 50B. Similarly to the HMI unit 12 described above, the HMI unit 51 includes an operation input device that performs various inputs to the determination device 50B and an information output device that performs various outputs from the determination device 50B.

The communication unit 52 is a communication module capable of communicating with devices other than the determination device 50B. The communication unit 52 has substantially the same configuration as the above-described communication unit 21, is communicably connected to the network NW regardless of wireless communication or wired communication, and communicates with other devices (for example, the data processing device 20, the mobility server 40A, the medical server 40B, the analysis device 50A, the user terminal 60, the medical institution terminal 70, and the service providing organization terminal 80) via the network NW.

The data input and output unit 53 is a data input and output device that inputs and outputs data (information) to and from the determination device 50B. The data input and output unit 53 has substantially the same configuration as the data input and output unit 14 described above, and is implemented by a recording medium interface or the like.

The determination storage unit 54B is a storage circuit that stores various kinds of data. The determination storage unit 54B has substantially the same configuration as the in-vehicle storage unit 15 described above, and is implemented by, for example, an HDD, an SSD, an optical disk, and a semiconductor memory. The determination storage unit 54B stores, for example, programs that enable units of the determination device 50B to implement various functions. The determination storage unit 54B stores various kinds of data necessary for various kinds of processes in the processing unit 55, and the processing unit 55 or the like reads the various kinds of data as necessary.

The processing unit 55 is a processing circuit that integrally controls the determination device 50B and implements various processing functions of the determination device 50B. The processing unit 55 has substantially the same configuration as the processing unit 16 described above, and is implemented by, for example, a processor, a RAM, and a ROM. The processing unit 55 implements processing functions, for example, by reading and executing the programs stored in the determination storage unit 54B. For example, the processing unit 55 is capable of executing a process of operating the HMI unit 51, a process of performing data communication via the communication unit 52, and the like.

The processing unit 55 according to the present embodiment includes the HMI processing unit 55a, the data processing unit 55b, and the communication processing unit 55c in order to implement the various processing functions of the determination device 50B. The processing unit 55 implements processing functions of the HMI processing unit 55a, the data processing unit 55b, and the communication processing unit 55c by reading and executing the program stored in the determination storage unit 54B, for example.

Similarly to the HMI processing unit 16b described above, the HMI processing unit 55a is a unit having a function capable of executing a process of controlling the HMI unit 51 and operating the HMI unit 51. The HMI processing unit 55a is capable of executing a process of controlling an operation input device constituting the HMI unit 51 and inputting an operation via the operation input device. The HMI processing unit 55a is capable of executing a process of controlling an information output device constituting the HMI unit 51 and outputting information via the information output device.

Similarly to the data processing unit 44b described above, the data processing unit 55b is a unit having a function capable of executing a process related to various kinds of data in the determination device 50B. The data processing unit 55b is capable of executing a process of storing data received via the communication unit 52 in the determination storage unit 54B. The data processing unit 55b is capable of executing a process of reading data transmitted via the communication unit 52 from the determination storage unit 54B. The data processing unit 55b is also capable of executing a process of storing, in the determination storage unit 54B, data input via the data input and output unit 53 and a process of reading data output via the data input and output unit 53 from the determination storage unit 54B. The data processing unit 55b is also capable of executing a process of storing, in the determination storage unit 54B, data registered in accordance with an operation on the operation input device constituting the HMI unit 51, and a process of reading the data from the determination storage unit 54B.

Similarly to the communication processing unit 44a described above, the communication processing unit 55c is a unit having a function capable of executing a process of controlling the communication unit 52 and performing data communication. The communication processing unit 55c is capable of executing a process of transmitting and receiving data to and from other devices such as the data processing device 20, the mobility server 40A, the medical server 40B, the analysis device 50A, the user terminal 60, the medical institution terminal 70, and the service providing organization terminal 80 via the communication unit 52.

The determination storage unit 54B of the determination device 50B includes a vehicle data storage unit 54a, a determination result data storage unit 54b, and a determination reference data storage unit 54c in terms of functional concept.

The vehicle data storage unit 54a is a storage area for storing the vehicle data D 1 acquired from the medical server 40B in the determination storage unit 54B. The vehicle data storage unit 54a stores the vehicle data D 1 including data related to the vehicle identification information D10, the DCM information D11, the vehicle basic information D12, the traveling information D13, and the like received from the data processing device 20.

The determination result data storage unit 54b is a storage area that stores, in the determination storage unit 54B, a determination result including the presence or absence of an abnormal indication of the user as the determination result data D4. The determination result data D4 typically includes the abnormal indication history information D41 which is information related to a history of determination for a certain period related to the presence or absence of an abnormal indication of the user

The determination reference data storage unit 54c is a storage area that stores, in the determination storage unit 54B, determination reference data D6, which is data related to a reference for determining the presence or absence, the content, and the like of an abnormal indication of the user from the vehicle data D1. The determination reference data D6 includes first reference information created based on a correlation relation between the driving operation in the vehicle V and the cognitive function state of the user, or second reference information indicating whether the driving operation corresponds to a predetermined driving operation which is an abnormal driving behavior, as a reference for determining the presence or absence, the content, or the like of an abnormal indication of the user. For example, the first reference information is information for determining that the user who drives the vehicle V has an indication of deterioration in cognitive function in a case where a day on which an occurrence frequency of an abnormal driving behavior performed by a person having an abnormal cognitive function exceeds a predetermined number of times continues for a predetermined period. Here, the "predetermined number of times" and the "predetermined period" are set to the number of times and the period that can be distinguished from a case where an abnormal driving behavior temporarily occurs, for example, a case where the user is forced to suddenly operate the steering wheel or suddenly brake due to a surrounding situation of the vehicle V or a case where the user intentionally takes an abnormal driving behavior for a short time. The second reference information is information on whether the driving operation of the user of the vehicle V corresponds to an abnormal driving behavior, and is reference information that can determine the presence or absence, the content, or the like of an abnormal indication of the user but cannot determine whether there is an indication of deterioration in cognitive function of the user.

The processing unit 55 of the determination device 50B further includes a determination processing unit 55e in addition to the HMI processing unit 55a, the data processing unit 55b, and the communication processing unit 55c described above in terms of functional concept.

The determination processing unit 55e is a unit having a function capable of executing a process of determining the presence or absence, the content, or the like of an abnormal indication of the user from the vehicle data D1 stored in the vehicle data storage unit 54a according to a predetermined cycle or an operation on the operation input device constituting the HMI unit 51. Here, the determination processing unit 55e is implemented as a unit that reads the vehicle data D1 and the determination reference data D6 from the determination storage unit 54B and determines the presence or absence, the content, and the like of an abnormal indication of the user from the vehicle data D1 based on the determination reference data D6.

The communication processing unit 55c of the determination device 50B is capable of executing a process of acquiring the vehicle data D1 from the medical server 40B via the communication unit 52. The communication processing unit 55c is configured to store the acquired vehicle data D1 in the vehicle data storage unit 54a. The communication processing unit 55c transmits the vehicle data D1 and the determination result data D4 to the medical institution terminal 70 and the service providing organization terminal 80. When there is an access request from the service providing organization terminal 80, it is possible to access the service providing organization terminal 80 so that the vehicle data D1 and the determination result data D4 can be acquired and browsed. The determination storage unit 54B of the determination device 50B does not store information (the user data D2) capable of directly specifying the user himself or herself, and even if the service providing organization terminal 80 accesses the vehicle data D1 or the determination result data D4, a user of the service providing organization terminal 80 cannot directly specify the user himself or herself from the data.

The data processing unit 55b of the determination device 50B is capable of executing a process of storing data related to the determination result generated by the determination processing unit 55e as the determination result data D4 in the determination storage unit 54B. The HMI processing unit 55a of the determination device 50B is capable of executing a process of controlling the information output device constituting the HMI unit 51 and outputting the determination result data D4 such as a determination result obtained by the determination processing unit 55e via the information output device.

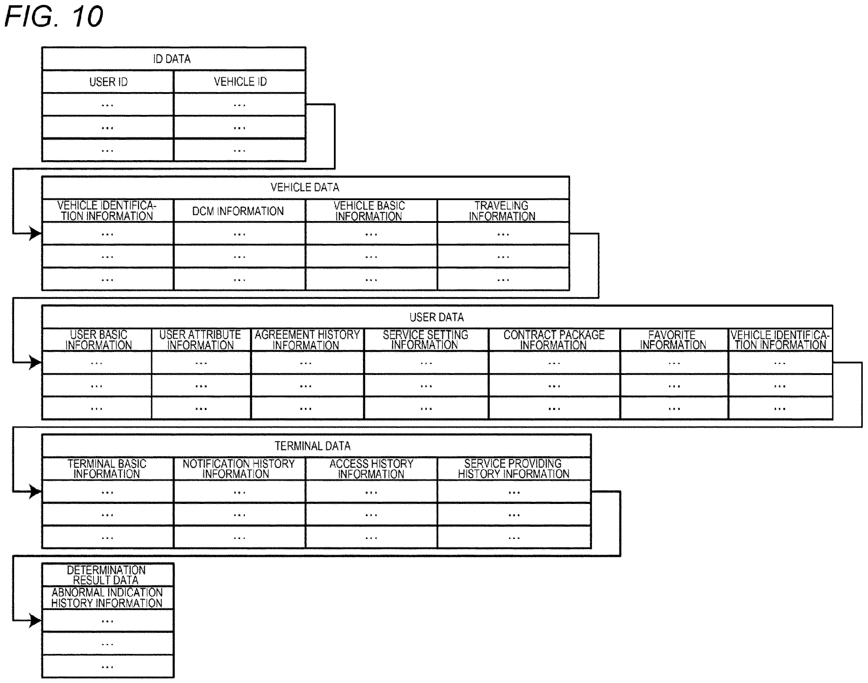

FIG. 10 is an example of a database stored in the mobility server 40A and the medical server 40B that constitute the storage unit of the consultation recommendation system 1. In the present embodiment, the mobility server 40A and the medical server 40B cooperate with each other to store the vehicle data D1, the user data D2, the terminal data D3, the determination result data D4, and the ID data D5 in association with each other as a database in a table format.

### <Basic Configuration of User Terminal>

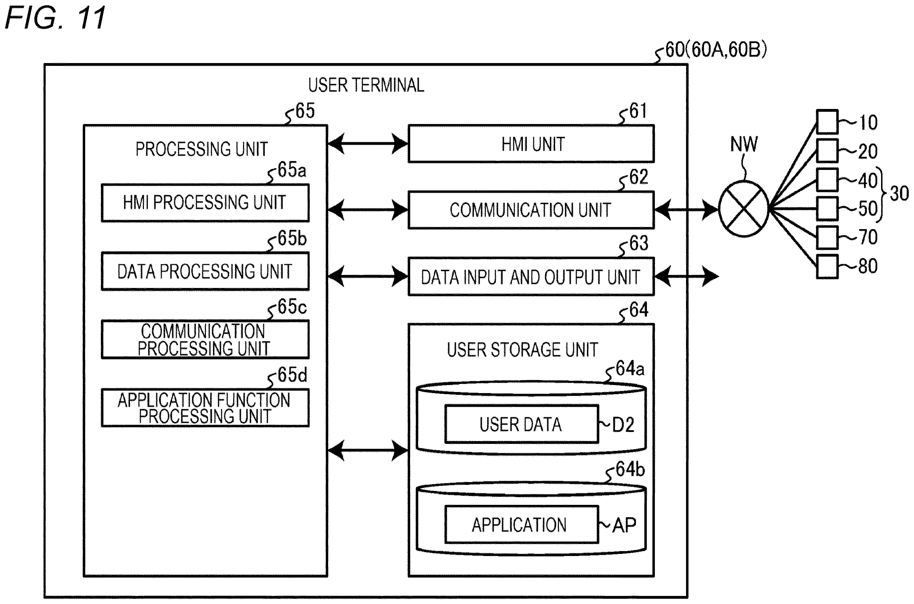

As described above, the user terminal 60 shown in FIG. 11 is a terminal device configured to receive information related to the consultation recommendation generated by the determination device 50B. The user terminal 60 includes the first user terminal 60A that is mainly owned and used by the user, and the second user terminal 60B that is mainly owned and used by a family of the user, a related person, or the like. Here, there is no substantial difference in configuration between the first user terminal 60A and the second user terminal 60B. The user terminal 60 may be configured to receive various services (for example, contract management, parking position search, vehicle remote control, emergency notification support, trouble support, security notification, and driving history evaluation) based on data stored in the mobility server 40A and the medical server 40B.

Specifically, the user terminal 60 includes an HMI unit 61, a communication unit 62, a data input and output unit 63, a user storage unit 64, and a processing unit 65. The HMI unit 61, the communication unit 62, the data input and output unit 63, the user storage unit 64, and the processing unit 65 are communicably connected to each other.

The HMI unit 61 is a device that performs various inputs and outputs in the user terminal 60. Similarly to the HMI unit 12 described above, the HMI unit 61 includes an operation input device serving as an input unit that performs various inputs to the user terminal 60, and an information output device serving as an output unit that performs various outputs from the user terminal 60.

The communication unit 62 is a communication module capable of communicating with devices other than the determination device 50B. The communication unit 62 has substantially the same configuration as the above-described communication unit 41, is communicably connected to the network NW regardless of wireless communication or wired communication, and communicates with other devices (for example, the data processing device 20, the mobility server 40A, the medical server 40B, the medical institution terminal 70, and the service providing organization terminal 80) via the network NW.

The data input and output unit 63 is a data input and output device that inputs and outputs data (information) to and from the user terminal 60. The data input and output unit 63 has substantially the same configuration as the data input and output unit 14 described above, and is implemented by a recording medium interface or the like.

The user storage unit 64 is a storage circuit that stores various kinds of data. The user storage unit 64 has substantially the same configuration as the in-vehicle storage unit 15 described above, and is implemented by, for example, an HDD, an SSD, an optical disk, and a semiconductor memory. The user storage unit 64 stores, for example, programs that enable units of the user terminal 60 to implement various functions. The user storage unit 64 stores various kinds of data necessary for various kinds of processes in the processing unit 65, and the processing unit 65 or the like reads the various kinds of data as necessary.

The processing unit 65 is a processing circuit that integrally controls the user terminal 60 and implements various processing functions of the user terminal 60. The processing unit 65 has substantially the same configuration as the processing unit 16 described above, and is implemented by, for example, a processor, a RAM, and a ROM. The processing unit 65 implements processing functions, for example, by reading and executing the programs stored in the user storage unit 64. For example, the processing unit 65 is capable of executing a process of operating the HMI unit 61, a process of performing data communication via the communication unit 62, and the like.

The processing unit 65 according to the present embodiment includes an HMI processing unit 65a, a data processing unit 65b, and a communication processing unit 65c in order to implement various processing functions of the user terminal 60. The processing unit 65 implements processing functions of the HMI processing unit 65a, the data processing unit 65b, and the communication processing unit 65c by reading and executing the program stored in the user storage unit 64, for example.

Similarly to the HMI processing unit 16b described above, the HMI processing unit 65a is a unit having a function capable of executing a process of controlling the HMI unit 61 and operating the HMI unit 61. The HMI processing unit 65a is capable of executing a process of controlling an operation input device constituting the HMI unit 61 and inputting an operation via the operation input device. The HMI processing unit 65a is capable of executing a process of controlling the information output device constituting the HMI unit 61 and outputting information via the information output device.

Similarly to the data processing unit 44b described above, the data processing unit 65b is a unit having a function capable of executing a process related to various kinds of data in the user terminal 60. The data processing unit 65b is capable of executing a process of storing data received via the communication unit 62 in the user storage unit 64. The data processing unit 65b is capable of executing a process of reading data transmitted via the communication unit 62 from the user storage unit 64. The data processing unit 65b is also capable of executing a process of storing data input via the data input and output unit 63 in the user storage unit 64 and a process of reading data output via the data input and output unit 63 from the user storage unit 64. The data processing unit 65b is also capable of executing a process of storing registered data in the user storage unit 64 in accordance with an operation on the operation input device constituting the HMI unit 61, and a process of reading the data from the user storage unit 64.

Similarly to the communication processing unit 44a described above, the communication processing unit 65c is a unit having a function capable of executing a process of controlling the communication unit 62 and performing data communication. The communication processing unit 65c is capable of executing a process of transmitting and receiving data to and from other devices such as the data processing device 20, the mobility server 40A, the medical server 40B, the medical institution terminal 70, and the service providing organization terminal 80 via the communication unit 62.

The user storage unit 64 of the user terminal 60 includes a user data storage unit 64a and an application storage unit 64b in terms of functional concept.

The user data storage unit 64a is a storage area for storing the user data D2 to be transmitted to the mobility server 40A in the user storage unit 64. The user data storage unit 64a stores, for example, the user data D2 including data related to the user basic information D20, the service setting information D23, the favorite information D25, and the like registered in accordance with an operation of the user on the operation input device constituting the HMI unit 61 of the first user terminal 60A.

The application storage unit 64b is a storage area for storing an application program AP used in the user terminal 60 in the user storage unit 64. The application program AP stored in the application storage unit 64b is executed in the user terminal 60, so that the notification of the consultation recommendation transmitted from the determination device 50B can be received. Here, an output unit of the HMI unit 61 includes a display unit having a touch panel function capable of inputting and outputting an operation by the user. In the present embodiment, the notification of the consultation recommendation includes presentation of a behavior recommended to the user according to the determination result data D4. When there is a notification of the consultation recommendation, the output unit of the HMI unit 61 presents, to the user, a behavior recommended to the user. Specifically, in response to receiving the consultation recommendation notification, the user terminal 60 executes the application program AP, and displays the behavior recommended to the user on the display unit of the HMI unit 61 according to the determination result data D4. According to this configuration, the behavior recommended to the user according to the determination result data D4 is presented to the user. When the input unit of the HMI unit 61 is operated and an instruction to execute the behavior recommended to the user is received, the user terminal 60 can operate the application program AP to execute the behavior recommended to the user. The application program AP is not limited to one that executes a process corresponding to the notification of the consultation recommendation, and may be capable of executing application functions corresponding to various services linked with the mobility server 40A.

The processing unit 65 of the user terminal 60 includes the HMI processing unit 65a, the data processing unit 65b, the communication processing unit 65c, and an application function processing unit 65d in terms of functional concept.

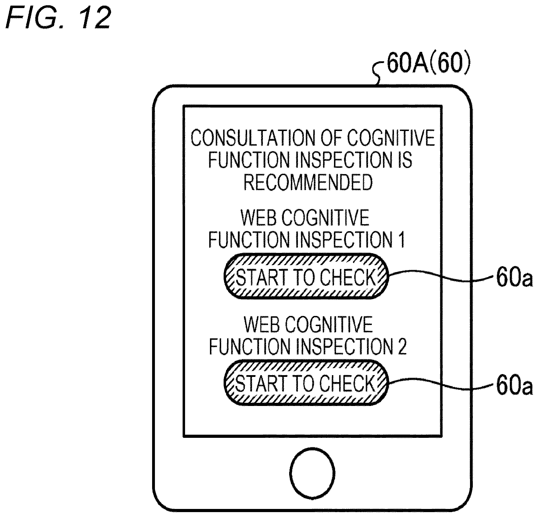

The HMI processing unit 65a of the user terminal 60 is capable of executing a process of controlling the display unit constituting the HMI unit 61, and displaying information and data related to various services executed by the application program AP on the display unit. FIGS. 12 to 14 illustrate an example of the "behavior recommended to the user according to the determination result data D4" displayed on the display unit of the HMI unit 61 when the user terminal 60 receives the notification of the consultation recommendation.

For example, as shown in FIG. 12, the display unit of the HMI unit 61 displays a message "a consultation of a cognitive function inspection is recommended" as the behavior recommended to the user, and displays an icon 60a for starting the consultation of the cognitive function inspection as the input unit of the HMI unit 61 in order for the user to execute the behavior. The user can consult the cognitive function inspection by touching this icon 60a. A plurality of behaviors recommended to the user may be displayed on the display unit of the HMI unit 61. In the example shown in FIG. 12, two kinds of WEB cognitive function inspections (WEB cognitive function inspection 1 and WEB cognitive function inspection 2) having different contents are selectably displayed for the user.

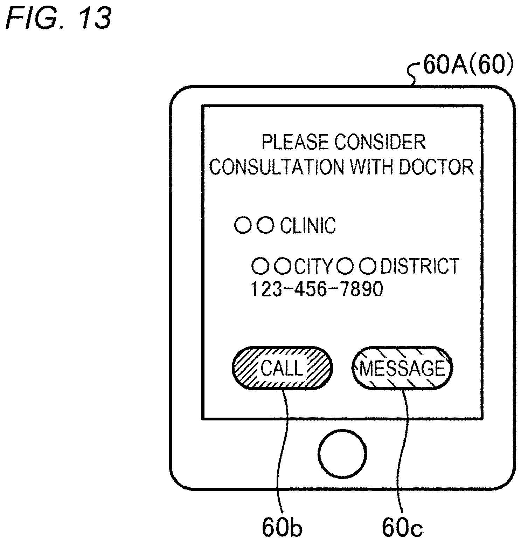

For example, as shown in FIG. 13, the display unit of the HMI unit 61 may display a message "please consider a consultation with a doctor" as the behavior recommended to the user, and display a call icon 60b that allows the user to make a call to the doctor, and a send icon 60c that allows the user to send a message to the doctor. In this case, when the user terminal 60 can make a phone call to the doctor when receiving a touch operation on the call icon 60b from the user. The user terminal 60 can send a message to the doctor in response to receiving a touch operation on the send icon 60c from the user

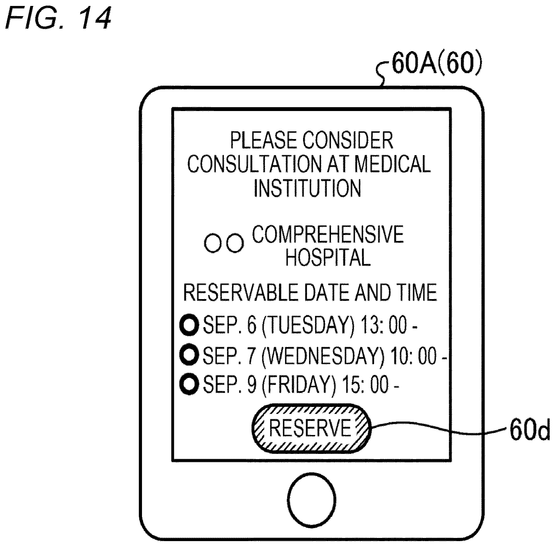

For example, as shown in FIG. 14, the display unit of the HMI unit 61 may display a massage "please consider a consultation at a medical institution" as the behavior recommended to the user, and display a reservation icon 60d for making a reservation for consultation at the medical institution. In this case, the user terminal 60 can make a reservation for consultation in the medical institution in response to receiving a touch operation on the reservation icon 60d from the user

The first user terminal 60A owned by the user himself or herself and the second user terminal 60B owned by the family of the user, the related person, or the like may be configured to be notified of the same content, or may be configured to be notified of different contents between the content for the user himself or herself and the content for the family or the related person. For example, when the notification of the consultation recommendation including a notification related to the cognitive function state of the user is issued, the same content may be notified to the first user terminal 60A and the second user terminal 60B, and the family of the user, the related person, or the like may also know the cognitive function state of the user. When different contents are notified between the content for the user himself or herself and the content for the family member, the related person, or the like, the notification of the consultation recommendation as illustrated in FIGS. 12 to 14 described above may be issued on the first user terminal 60A as the content for the user himself or herself, and a notification related to a simple contact indicating that the user is notified of the consultation recommendation to the medical institution may be issued to the second user terminal 60B as the content for the family member, the related person, or the like.

The data processing unit 65b of the user terminal 60 is capable of executing a process related to a behavior recommended to the user presented on a display unit of the user terminal 60 in accordance with an operation on the operation input device constituting the HMI unit 61. The data processing unit 65b of the user terminal 60 is capable of executing a process of storing, in the user data storage unit 64a as the user data D2, data related to the user basic information D20, the service setting information D23, the favorite information D25, and the like registered in accordance with an operation on the operation input device constituting the HMI unit 61. The data processing unit 65b of the user terminal 60 is capable of executing a process of reading the user data D2 stored in the user data storage unit 64a in response to a request.

The communication processing unit 65c of the user terminal 60 is capable of executing a process of transmitting the user data D2 stored in the user data storage unit 64a to the mobility server 40A via the communication unit 62. The communication processing unit 65c of the user terminal 60 is capable of executing a process of transmitting various commands and requests to the mobility server 40A, the medical server 40B, and the like via the communication unit 62. The communication processing unit 65c of the user terminal 60 is capable of executing a process of receiving various commands and requests to the mobility server 40A, the medical server 40B, and the like via the communication unit 62. The communication processing unit 65c of the user terminal 60 is capable of executing a process of receiving various kinds of information according to a service content and an application function from the mobility server 40A, the medical server 40B, the determination device 50B, or the like via the communication unit 62. The data received by the communication processing unit 65c of the user terminal 60 from the mobility server 40A, the medical server 40B, the determination device 50B, or the like according to the service content or the application function may include, for example, a notification of a consultation recommendation, the vehicle data D1, the user data D2, and the determination result data D4 stored in the mobility server 40A and the medical server 40B, and various kinds of information, data, and the like generated based on the data.

The application function processing unit 65d is a unit having a function capable of executing a process of executing the application program AP stored in the application storage unit 64b. The application function processing unit 65d activates the application program AP stored in the application storage unit 64b in response to, for example, receiving the consultation recommendation notification from the determination device 50B or an operation performed on the operation input device constituting the HMI unit 61, and is capable of executing application functions corresponding to various services.

### <Basic Configuration of Medical Institution Terminal>

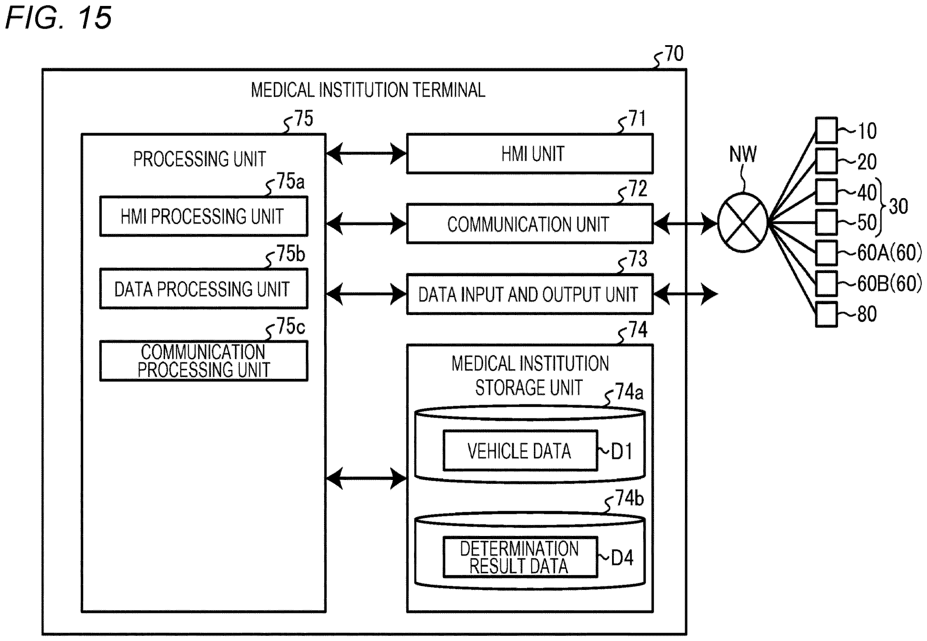

As described above, the medical institution terminal 70 shown in FIG. 15 is configured to be accessible to the determination device 50B, and is a device mainly used by a medical worker such as a doctor. Typically, the medical institution terminal 70 is a terminal device mainly provided at a medical institution. The medical institution terminal 70 is configured to receive the vehicle data D1 and the determination result data D4 transmitted from the determination device 50B and store the data in the storage unit. The medical institution terminal 70 makes an access request to the determination device 50B, and is accessible to the vehicle data D1 and the determination result data D4 when the access request is permitted.

Specifically, the medical institution terminal 70 includes an HMI unit 71, a communication unit 72, a data input and output unit 73, a medical institution storage unit 74, and a processing unit 75. The HMI unit 71, the communication unit 72, the data input and output unit 73, the medical institution storage unit 74, and the processing unit 75 are communicably connected to each other.

The HMI unit 71 is a device that performs various inputs and outputs in the medical institution terminal 70. Similarly to the HMI unit 12 described above, the HMI unit 81 includes an operation input device that performs various inputs to the medical institution terminal 70 and an information output device that performs various outputs from the medical institution terminal 70.

The communication unit 72 is a communication module capable of communicating with devices other than the medical institution terminal 70. The communication unit 72 has substantially the same configuration as the communication unit 21 described above, is communicably connected to the network NW regardless of wireless communication or wired communication, and communicates with other devices (for example, the service providing device 30, the mobility server 40A, the medical server 40B, the analysis device 50A, and the determination device 50B) via the network NW.

The data input and output unit 73 is a data input and output device that inputs and outputs data (information) to and from the medical institution terminal 70. The medical institution terminal 70 has substantially the same configuration as the data input and output unit 14 described above, and is implemented by a recording medium interface or the like.

The medical institution storage unit 74 of the medical institution terminal 70 includes a vehicle data storage unit 74a and a determination result data storage unit 74b in terms of functional concept.

The vehicle data storage unit 74a of the medical institution terminal 70 is a storage area for storing the vehicle data D1 in the medical institution terminal 70. The vehicle data storage unit 74a stores the vehicle data D1 transmitted from the determination device 50B. The determination result data storage unit 74b of the medical institution terminal 70 is a storage area for storing the determination result data D4 in the medical institution terminal 70. The determination result data storage unit 74b stores the determination result data D4 transmitted from the determination device 50B.

The processing unit 75 of the medical institution terminal 70 includes an HMI processing unit 75a, a data processing unit 75b, and a communication processing unit 75c in terms of functional concept.

The communication processing unit 75c of the medical institution terminal 70 is capable of executing a process of transmitting an access request to the determination device 50B via the communication unit 72. For example, the communication processing unit 75c transmits an access request to the determination device 50B in response to an operation on an operation input device constituting the HMI unit 71.

### <Basic Configuration of Service Providing Organization Terminal>

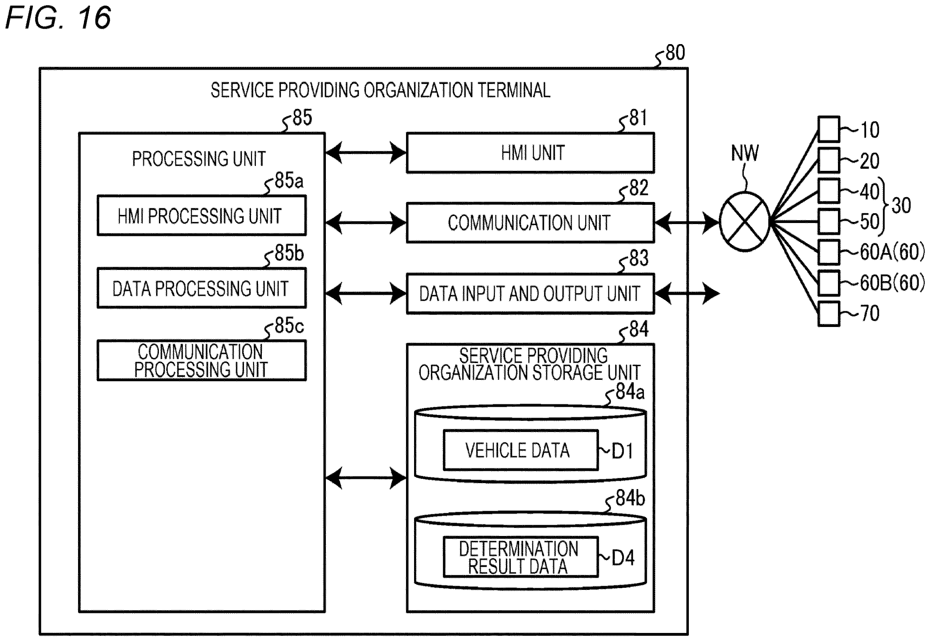

The service providing organization terminal 80 shown in FIG. 16 is accessible to the determination device 50B, and is a device mainly used by an employee of a service providing organization such as a pharmaceutical manufacturer or an insurance company. Typically, the service providing organization terminal 80 is a terminal device mainly installed in the service providing organization. The service providing organization terminal 80 is capable of receiving and storing the vehicle data D1 and the determination result data D4 transmitted from the determination device 50B. The service providing organization terminal 80 makes an access request to the determination device 50B, and is accessible to the vehicle data D1 and the determination result data D4 when the access request is permitted.

Specifically, the service providing organization terminal 80 includes an HMI unit 81, a communication unit 82, a data input and output unit 83, a service providing organization storage unit 84, and a processing unit 85. The HMI unit 81, the communication unit 82, the data input and output unit 83, the service providing organization storage unit 84, and the processing unit 85 are communicably connected to each other.

The HMI unit 81 is a device that performs various inputs and outputs in the service providing organization terminal 80. Similarly to the HMI unit 12 described above, the HMI unit 81 includes an operation input device that performs various inputs to the service providing organization terminal 80 and an information output device that performs various outputs from the service providing organization terminal 80.

The communication unit 82 is a communication module capable of communicating with devices other than the service providing organization terminal 80. The communication unit 82 has substantially the same configuration as the communication unit 21 described above, is communicably connected to the network NW regardless of wireless communication or wired communication, and communicates with other devices (for example, the service providing device 30, the mobility server 40A, the medical server 40B, the analysis device 50A, and the determination device 50B) via the network NW.

The data input and output unit 83 is a data input and output device that inputs and outputs data (information) to and from the service providing organization terminal 80. The data input and output unit 83 has substantially the same configuration as the data input and output unit 14 described above, and is implemented by a recording medium interface or the like.

The service providing organization storage unit 84 of the service providing organization terminal 80 includes a vehicle data storage unit 84a and a determination result data storage unit 84b in terms of functional concept.

The vehicle data storage unit 84a of the service providing organization terminal 80 is a storage area for storing the vehicle data D1 in the service providing organization terminal 80. The vehicle data storage unit 84a stores the vehicle data D1 transmitted from the determination device 50B. The determination result data storage unit 84b of the service providing organization terminal 80 is a storage area for storing the determination result data D4 in the service providing organization terminal 80. The determination result data storage unit 84b stores the determination result data D4 transmitted from the determination device 50B.

The processing unit 85 of the service providing organization terminal 80 includes an HMI processing unit 85a, a data processing unit 85b, and a communication processing unit 85c in terms of functional concept.

The communication processing unit 85c of the service providing organization terminal 80 is capable of executing a process of transmitting an access request to the determination device 50B via the communication unit 82. For example, the communication processing unit 85c transmits an access request to the determination device 50B in response to an operation on an operation input device constituting the HMI unit 81.

An outline of an overall configuration of the consultation recommendation system 1 according to the present embodiment has been described above.

### <Flow of Data in Consultation Recommendation System>

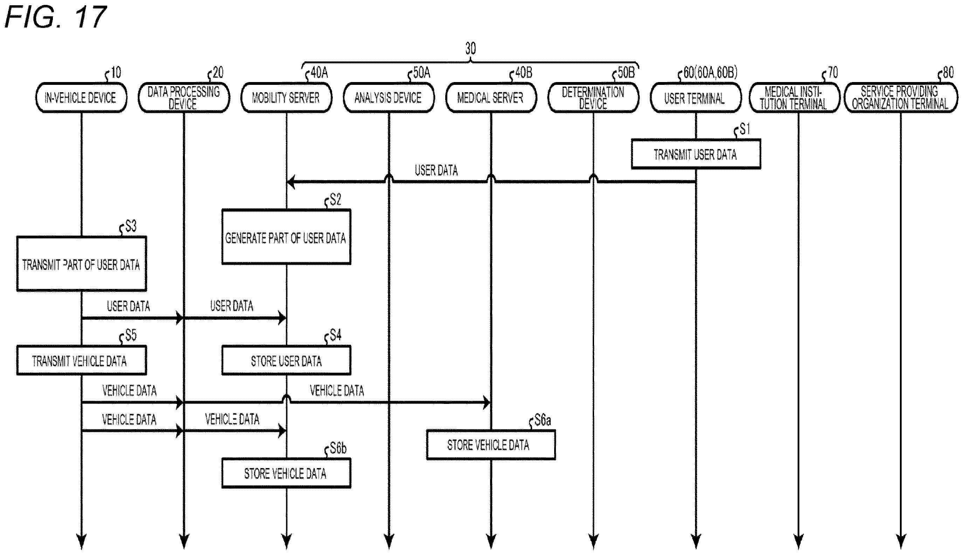

Next, an example of a flow of each process in the consultation recommendation system 1 according to the present embodiment will be described with reference to FIGS. 17 to 19. In the following description, transmission and reception of data are performed via the network NW, but the description thereof may be omitted. In the following description, in order to mainly focus on the flow of data, a description of specific processing operations related to the processing unit and the like in each device may be omitted as appropriate, and the outline of the entire configuration described above will be referred to as necessary. The flow of each process described below is merely an example of a representative process, and the present invention is not limited thereto.

### <Storage of User Data and Vehicle Data>

First, as shown in FIG. 17, the user terminal 60 (the first user terminal 60A) transmits, as the user data D2 to the mobility server 40A, data related to the user basic information D20, the service setting information D23, the favorite information D25, and the like registered in the user terminal 60 (the first user terminal 60A) in the user data D2 (step S1), for example. The user terminal 60 (the first user terminal 60A) is capable of transmitting not only the initially registered user data D2 but also the user data D2 updated after the initial registration to the mobility server 40A. The user terminal 60 (the first user terminal 60A) can transmit, for example, the user basic information D20 including the terminal registration information of the second user terminal 60B as the user data D2 such that the consultation recommendation notification for the user is also notified to the second user terminal 60B mainly used by the family of the user, the related person, or the like. The user terminal 60 (the first user terminal 60A) may transmit the user data D2 including the vehicle identification information D10 to the mobility server. In a case where the user newly registers a service related to the notification of the consultation recommendation, in a case where the registration information is changed for the service related to the notification of the consultation recommendation that has already been registered, or the like, it is possible to register the vehicle identification information D10 in the user data D2 by including the vehicle identification information D10 in the terminal registration information using the user terminal 60 (the first user terminal 60A). Accordingly, even when the vehicle data D1 is not stored in the mobility server 40A, the user data D2 and the determination result data D4 can be managed in association with each other based on the vehicle identification information D10.

In response to receiving the user data D2 from the user terminal 60 (the first user terminal 60A), the mobility server 40A automatically generates a part of the user data D2 based on the received user data D2 (step S2). Here, the mobility server 40A automatically generates data related to the user attribute information D21, the agreement history information D22, the contract package information D24, and the like based on data related to the user basic information D20, the service setting information D23, the favorite information D25, and the like received from the user terminal 60 (the first user terminal 60A), for example.

The user data D2 may be registered, for example, via the in-vehicle device 10. In this case, the in-vehicle device 10 can register a part of the information of the user data D2 in accordance with the operation on the HMI unit 12 performed by the user, and also can transmit data related to the registered part of the information as the user data D2 to the data processing device 20, and transmit the data to the mobility server 40A via the data processing device 20 (step S3).

The mobility server 40A stores the received user data D2 and the automatically generated user data D2 in the server storage unit 43 (the user data storage unit 43c) in association with the user ID D50 (step S4).

The in-vehicle device 10 transmits the vehicle data D1 detected or acquired by the detection plant acquisition unit 11 to the data processing device 20 at an appropriate timing (step S5). In this case, the in-vehicle device 10 transmits the vehicle data D1 to the data processing device 20. In this case, the vehicle data D1 includes the vehicle identification information D10. In response to receiving the vehicle data D1 from the in-vehicle device 10, the data processing device 20 transmits the received vehicle data D1 to the mobility server 40A and the medical server 40B. Here, the vehicle data D1 transmitted to the medical server 40B is data used for determining the presence or absence, the content, or the like of an abnormal indication of the user, and may include at least information necessary for issuing a notification of a consultation recommendation. That is, the vehicle data D1 transmitted from the vehicle V to the medical server 40B need not be all the data transmitted by the in-vehicle device 10, and may be a part of the vehicle data D1 transmitted by the in-vehicle device 10. For example, the vehicle data D1 transmitted from the vehicle V to the mobility server 40A may include the vehicle identification information D10, the DCM information D11, the vehicle basic information D12, and the traveling information D13, and the vehicle data D1 transmitted to the medical server 40B may include only the vehicle identification information D10 and the traveling information D13. The traveling information D13 included in the vehicle data D1 transmitted to the medical server 40B can effectively use an amount of data communication and a storage resource while improving the secret retention of data, by limiting only data used in the determination of the presence or absence, the content, or the like of an abnormal indication of the user, such as data related to a vehicle position, a vehicle speed, acceleration/deceleration, a steering wheel steering angle, an accelerator opening degree (an accelerator operation amount), and a brake opening degree (an accelerator operation amount).

In response to receiving the vehicle data D1 from the data processing device 20, the medical server 40B stores the vehicle data D1 in the server storage unit 43 (the vehicle data storage unit 43a) of the medical server 40B (step S6a). In response to receiving the vehicle data D1 from the data processing device 20, the mobility server 40A stores the vehicle data D1 in the server storage unit 43 (the vehicle data storage unit 43a) of the mobility server 40A (step S6b).

### <Determination of Abnormal Indication of User and Notification of Consultation Recommendation>

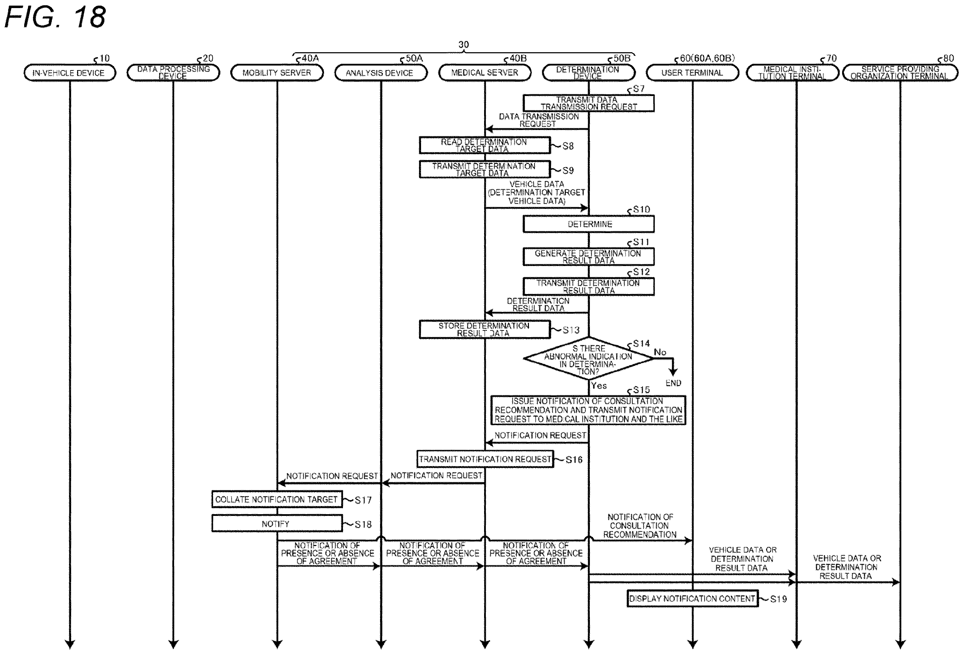

As shown in FIG. 18, in response to acquiring the vehicle data D1 used to determine an abnormal indication of the user from the medical server 40B, the determination device 50B transmits a data transmission request to the medical server 40B (step S7). In response to receiving the data transmission request from the determination device 50B, the medical server 40B reads the vehicle data D1 to be determined (step S8). Then, the medical server 40B transmits the read vehicle data D1 (vehicle data to be determined) to the determination device 50B (step S9). In response to receiving the vehicle data D1, the determination device 50B determines the presence or absence of an abnormal indication of the user who drives the vehicle V from the vehicle data D1 (step S10). In this case, the determination processing unit 55e of the determination device 50B determines the presence or absence of an abnormal indication of the user from the vehicle data D1 based on the determination reference data D6. Here, the abnormal indication of the user includes an indication of deterioration in cognitive function of the user determined based on the determination reference data D6, an abnormality of a driving behavior of the user detected based on the determination reference data D6, and the like.

Thereafter, the determination device 50B generates a determination result as the determination result data D4 and stores the generated determination result in the determination storage unit 54B (step S 11). In the determination device 50B, the determination result data D4 is managed in association with the vehicle identification information D10 included in the vehicle data D1 corresponding to the determination target. The determination device 50B transmits the determination result data D4 in association with the vehicle identification information D10 to the medical server 40B (step S12). In response to receiving the determination result data D4, the medical server 40B stores the determination result data D4 in association with the vehicle identification information D10 in the server storage unit 43 (the determination result data storage unit 43e) and manages the determination result data D4 (step S13). The determination device 50B confirms whether the determination result is a result that there is an abnormal indication in the user who drives the vehicle V (step S14). Here, when the determination result is a result that there is no abnormal indication in the user who drives the vehicle V, the determination device 50B ends the process until the next determination is performed. On the other hand, when the determination result is a result that there is an abnormal indication in the user who drives the vehicle V, the determination device 50B issues a notification of a consultation recommendation to the user to be determined (step S 15). Here, the determination device 50B may issue a notification to a medical institution or a service providing organization together with the notification of the consultation recommendation. The notification of the consultation recommendation is issued by the determination device 50B transmitting a notification request to the medical server 40B. Here, the notification request requests the user corresponding to the vehicle identification information D10 associated with the determination result data D4 to be issued the notification of the consultation recommendation. The notification request includes the vehicle identification information D10 usable for specifying the user. When the notification request is made, the determination device 50B may transmit the determination result data D4 in step S12 simultaneously with the transmission of the notification request. A notification to the medical institution or the service providing organization is issued by the determination device 50B transmitting the vehicle data D1 or the determination result data D4 to the medical institution or the service providing organization. Here, when the vehicle data D1 or the determination result data D4 is to be transmitted to the medical institution or the service providing organization, the determination device 50B performs, on the mobility server 40A via the medical server 40B and the analysis device 50A, agreement confirmation as to whether the user agrees to provide the vehicle data D1 or the determination result data D4 to the medical institution or the service providing organization. The agreement confirmation is included in the notification request from the determination device 50B to the medical server 40B.

In response to receiving the notification request from the determination device 50B, the medical server 40B transmits a notification request to the mobility server 40A via the analysis device 50A so as to performs collation on a notification target (step S16). In response to receiving the notification request from the medical server 40B via the analysis device 50A, the mobility server 40A performs collation on a notification target of the consultation recommendation (step S17). In the collation of the notification target, the vehicle identification information D10 included in the vehicle data D1 and the user data D2 are stored in association with each other, and the vehicle identification information D10 included in the vehicle data D1 and the determination result data D4 are stored in association with each other, and thus the mobility server 40A specifies the user terminal 60 from the user data D2 corresponding to the determination result data D4 based on the vehicle identification information D10 included in the vehicle data D1 used in the determination by the determination device 50B, and performs collation on the notification target of the consultation recommendation. Specifically, the determination device 50B transmits a notification request including the vehicle identification information D10 corresponding to the notification target of the consultation recommendation to the medical server 40B, and the medical server 40B transmits the received notification request to the mobility server 40A via the analysis device 50A. The mobility server 40A specifies the user data D2 corresponding to the notification request and the determination result data D4 by collating the vehicle identification information D10 included in the notification request with the vehicle identification information D10 associated with the user data D2. The mobility server 40A specifies the user terminal 60 to which the notification of the consultation recommendation is transmitted, based on the terminal registration information of the user terminal 60 included in the service setting information D23 of the specified user data D2. The mobility server 40A performs agreement confirmation as to whether the user agrees to provide the vehicle data D1 or the determination result data D4 to the medical institution or the service providing organization. As described above, the mobility server 40A specifies the user of a notification target of the consultation recommendation from the service setting information D23 included in the user data D2 by collating the vehicle identification information D10 included in the notification request and the vehicle identification information D10 associated with the user data D2. Further, the mobility server 40A can confirm whether the user agrees to provide the vehicle data D1 or the determination result data D4 to the medical institution or the service providing organization from the agreement history information D22 included in the user data D2.

Thereafter, the mobility server 40A issues, to the user, a notification of the consultation recommendation by transmitting information related to the consultation recommendation to the specified user terminal 60 (step S18). The mobility server 40A transmits, to the determination device 50B, information on whether the user agrees to provide the vehicle data D1 or the determination result data D4 to the medical institution or the service providing organization. In response to receiving the information indicating that the user does not agree, the determination device 50B transmits the vehicle data D1 or the determination result data D4 to the medical institution or the service providing organization. Here, when transmitting the determination result data D4, the determination device 50B transmits the determination result data D4 in association with the vehicle identification information D10 included in the vehicle data D1. When the determination result data D4 is transmitted in association with the vehicle identification information D10, for example, in a case where the user who has received the notification of the consultation recommendation makes a consultation reservation from the user terminal 60 (the first user terminal 60A), the medical institution can associate the acquired determination result data D4 with the user who has made the consultation reservation based on the vehicle identification information D10. With this configuration, the medical institution can more accurately grasp the state of the user. In this case, the consultation reservation made by the user terminal 60 (the first user terminal 60A) includes the vehicle identification information D10. By transmitting the determination result data D4 in association with the vehicle identification information D10, for example, the service providing organization can provide the user with information related to various services via the service providing device 30. In response to receiving the information indicating that the user does not agree, the determination device 50B does not transmit the vehicle data D1 or the determination result data D4 to the medical institution or the service providing organization.

In response to receiving the information related to the consultation recommendation from the mobility server 40A, the user terminal 60 displays a notification content of the consultation recommendation on the display unit (step S19). For example, the user terminal 60 displays the behavior recommended to the user as shown in FIGS. 12 to 14 on the display unit according to the content of the determination result data D4.

### <Access from Service Providing Organization Terminal>

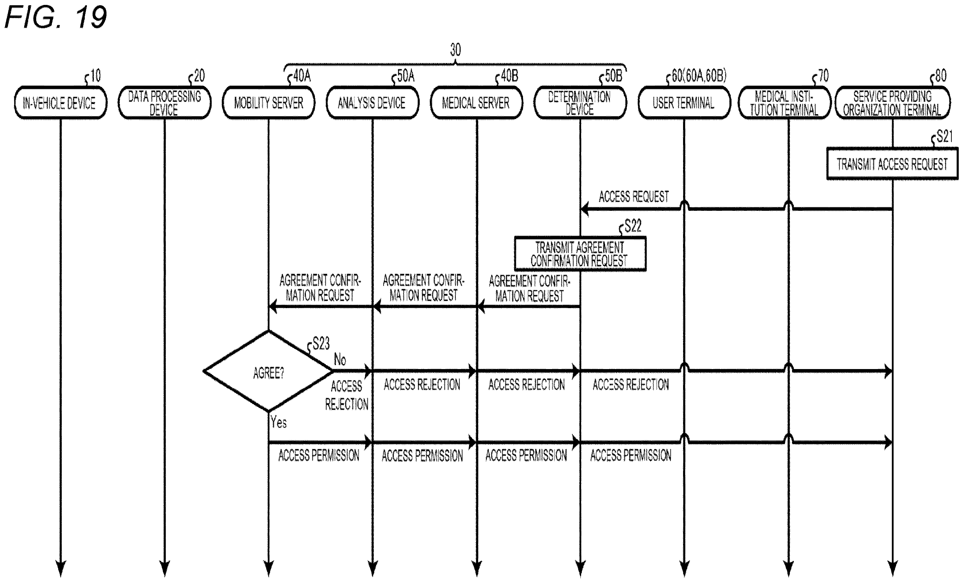

As shown in FIG. 19, the service providing organization terminal 80 can make an access request to the determination device 50B in order to use the vehicle data D1 and the determination result data D4 stored in the determination device 50B. First, when making the access request to the determination device 50B, the service providing organization terminal 80 transmits the access request to the determination device 50B (step S21). In response to receiving the access request, the determination device 50B transmits, to the mobility server 40A, an agreement confirmation request for confirming whether the user agrees with the use of the vehicle data D1 or the determination result data D4 by the service providing organization (step S22). Here, the agreement confirmation request is made via the medical server 40B and the analysis device 50A. The mobility server 40A confirms whether each user agrees with the use of the vehicle data D1 or the determination result data D4 by the service providing organization. The mobility server 40A permits the access of the service providing organization terminal 80 to the vehicle data D1 or the determination result data D4 of a user who agrees with the use of the vehicle data D1 or the determination result data D4 by the service providing organization. The mobility server 40A rejects the access of the service providing organization terminal 80 to the vehicle data D1 or the determination result data D4 of a user who does not agree with the use of the vehicle data D1 or the determination result data D4 by the service providing organization.

### <Effects of First Embodiment>

As described above, the consultation recommendation system 1 according to the present embodiment includes: the mobility server 40A that stores the user data D2 related to the user of the vehicle V; the medical server 40B that stores the vehicle data D1 including the traveling information D13 of the vehicle V; the determination device 50B that determines the presence or absence of an abnormal indication of the user from the vehicle data D1, stores the determination result data D4 including a determination result in the medical server 40B, and issues a notification related to a cognitive function to the user based on the determination result data D4; and the user terminals 60, 60A, and 60B that receive the notification from the determination device 50B, in which the mobility server 40A and the medical server 40B cooperate to manage the user data D2, the vehicle data D1, and the determination result data D4 in association with each other, and when the determination device 50B determines that there is an abnormal indication in the user, the determination device 50B specifies the user terminals 60, 60A, and 60B based on the user data D2 corresponding to the determination result, and transmits, to the user terminals 60, 60A, and 60B, a notification of a consultation recommendation on a disease related to the cognitive function.

The consultation recommendation system 1 according to the present embodiment determines the presence or absence of an abnormal indication of the user from the vehicle data D1 collected by the in-vehicle device 10, and thus it is possible to discover an indication, a symptom, or the like of deterioration in cognitive function at an early stage based on a driving situation of the vehicle V which is a daily behavior of the user. The determination of the presence or absence of the abnormal indication of the user in the consultation recommendation system 1 is not based on a temporary state of a person who receives the inspection, unlike simple inspection related to the cognitive function performed in the related art, and the determination of the presence or absence of the abnormal indication of the user is performed from the vehicle data D1 accumulated in the in-vehicle device 10 or the like. Accordingly, as compared with the simple inspection as described above, since an indication or a symptom of deterioration in cognitive function is more likely to appear in the determination result, and in particular, an abnormality in cognitive function in an initial stage is more likely to be detected, and thus it is possible to appropriately issue the notification of the consultation recommendation. By issuing, to the user terminal 60, the notification of the consultation recommendation on the disease related to the cognitive function, it is possible to improve the intention of the user to visit the medical institution. In the consultation recommendation system 1 according to the present embodiment, the user data D2 is managed by the mobility server 40A, and the determination result data D4, which is personal information having high confidentiality, is managed by the medical server 40B, and thus the determination result data D4 and the user data D2 are managed separately, so that it is possible to smoothly utilize data used for mobility services or various kinds of data used for medical services while improving secret retention.

In the consultation recommendation system 1 according to the present embodiment, the mobility server 40A is disposed in the mobility base station 101 that relays communication between the vehicle V and the user terminals 60, 60A, and 60B and manages the user data D2, and the medical server 40B is a base station different from the mobility base station 101, and is disposed in the medical base station 102 that relays communication between the vehicle V and the user terminals 60, 60A, and 60B and manages the vehicle data D1 and the determination result data D4.

In the consultation recommendation system 1 according to the present embodiment, the mobility server 40A and the medical server 40B are provided in different base stations (the mobility base station 101 and the medical base station 102). According to this configuration, it is possible to manage the data stored in the server according to the degree of confidentiality. For example, the medical institution treats the information related to the body, such as a medical record of a patient, and thus, higher information security is required in data management. The medical institution that requires high information security manages the medical base station 102, and the medical server 40B is disposed in the medical base station 102, thereby improving confidentiality of the determination result data D4. In this way, by classifying the server and the base station according to the type of data, appropriate information management can be performed.

In the consultation recommendation system 1 according to the present embodiment, the vehicle data D1 includes the vehicle identification information D10 for identifying the vehicle, the mobility server 40A further manages the vehicle data D1, stores the user data D2 and the vehicle identification information D10 in association with each other, the medical server 40B stores the determination result data D4 and the vehicle identification information D10 in association with each other, the determination device 50B specifies the user terminals 60, 60A, and 60B from the user data D2 corresponding to the determination result data D4 based on the vehicle identification information D10, and transmits a notification of a consultation recommendation to the user terminals 60, 60A, and 60B.

The user data D2 and the determination result data D4 are associated with each other via the vehicle identification information D10, and thus the user data D2 and the determination result data D4 having particularly high confidentiality can be used without being directly linked, and confidentiality of data management can be improved.

In the consultation recommendation system 1 according to the present embodiment, the notification of the consultation recommendation includes a presentation of a behavior recommended to the user, and the user terminals 60, 60A, and 60B include an output unit configured to present the behavior to the user and the input unit icons 60a to 60d configured to receive an operation of performing the behavior.

The consultation recommendation system 1 according to the present embodiment presents a specific behavior recommended to the user, and is provided with an input unit (the icons 60a to 60d) capable of receiving an operation of executing the behavior, so that it is possible to improve the intention of the user to visit the medical institution. Deterioration in cognitive function may not appear in an external wound, and for example, the user may not know which department the specialist corresponds to, or may be hard to visit a comprehensive hospital for an initial inspection. Even in such a case, the user terminal 60 displays the input unit (the icons 60a to 60d) in an operable manner, so that the user can smoothly take the recommended behavior. That is, by providing the input unit (the icons 60a to 60d) in the user terminal 60 in an operable manner, it is possible to reduce a psychological barrier when the user makes a consultation reservation at a medical institution or the like.

In the consultation recommendation system 1 according to the present embodiment, the traveling information D13 includes information related to the driving operation of the vehicle V, and the determination device 50B determines the presence or absence of an abnormal indication of the user based on the determination reference data D6 including the first reference information based on the correlation relation between the driving operation of the vehicle V and the cognitive function state of the user or the second reference information based on the determination as to whether the driving operation of the vehicle V corresponds to the predetermined driving operation.

The consultation recommendation system 1 according to the present embodiment determines the presence or absence of an abnormal indication of the user from the vehicle data D1 collected by the in-vehicle device 10, and thus it is possible to discover an indication or a symptom of deterioration in cognitive function at an early stage based on the driving situation of the vehicle V which is a daily behavior of the user.

In addition, in the consultation recommendation system 1 according to the present embodiment, the medical server 40B directly acquires the vehicle data D1 from outside without synchronizing with the mobility server 40A.

In the consultation recommendation system 1 according to the present embodiment, the medical server 40B directly acquires the vehicle data D1 from outside without synchronizing with the mobility server 40A, so that an information type, a reception circle, a data amount, and the like included in the vehicle data D1 can be configured in a format suitable for each server, without depending on the server, for example.

The consultation recommendation system 1 according to the present embodiment further includes the medical institution terminal 70 installed at a medical institution, and the determination device 50B is configured to transmit at least one of the determination result data D4 and the vehicle data D1 to the medical institution terminal 70.

The consultation recommendation system 1 according to the embodiment can enable the medical institution to use the vehicle data D1 and the determination result data D4 by transmitting the vehicle data D1 and the determination result data D4 from the determination device 50B to the medical institution terminal 70. Accordingly, when the user receives the consultation recommendation and receives the consultation at a medical institution, appropriate support can be appropriately received from the medical institution.

The base station device (the service providing device 30) according to the present embodiment includes: the mobility server 40A that stores the user data D2 related to the user of the vehicle V; the medical server 40B that stores the vehicle data D1 including the traveling information D13 of the vehicle V; and the determination device 50B that determines the presence or absence of an abnormal indication of the user from the vehicle data D1, stores the determination result data D4 including a determination result in the medical server 40B, and issues a notification related to a cognitive function to the user based on the determination result data D4, in which the mobility server 40A and the medical server 40B cooperate to manage the user data D2, the vehicle data D1, and the determination result data D4 in association with each other, and when the determination device 50B determines that there is an abnormal indication in the user, the determination device 50B transmits, to a user corresponding to the determination result, a notification of a consultation recommendation on a disease related to the cognitive function.

The base station device (the service providing device 30) according to the present embodiment determines the presence or absence of an abnormal indication of the user from the vehicle data D1 collected by the in-vehicle device 10, and thus it is possible to discover an indication, a symptom, or the like of deterioration in cognitive function at an early stage based on the driving situation of the vehicle V which is a daily behavior of the user. The determination of the presence or absence of the abnormal indication of the user in the consultation recommendation system 1 is not based on a temporary state of a person who receives the inspection, unlike simple inspection related to the cognitive function performed in the related art, and the determination of the presence or absence of the abnormal indication of the user is performed from the vehicle data D1 accumulated in the in-vehicle device 10 or the like. Accordingly, as compared with the simple inspection as described above, since an indication or a symptom of deterioration in cognitive function is more likely to appear in the determination result, and in particular, an abnormality in cognitive function in an initial stage is more likely to be detected, and thus it is possible to appropriately issue the notification of the consultation recommendation. By issuing, to the user terminal 60, the notification of the consultation recommendation on the disease related to the cognitive function, it is possible to improve the intention of the user to visit the medical institution. In the base station device (the service providing device 30) according to the present embodiment, the user data D2 is managed by the mobility server 40A, and the determination result data D4, which is personal information having high confidentiality, is managed by the medical server 40B, and thus the determination result data D4 and the user data D2 are managed separately, so that it is possible to smoothly utilize data used for mobility services or various kinds of data used for medical services while improving secret retention.

### <Second Embodiment>

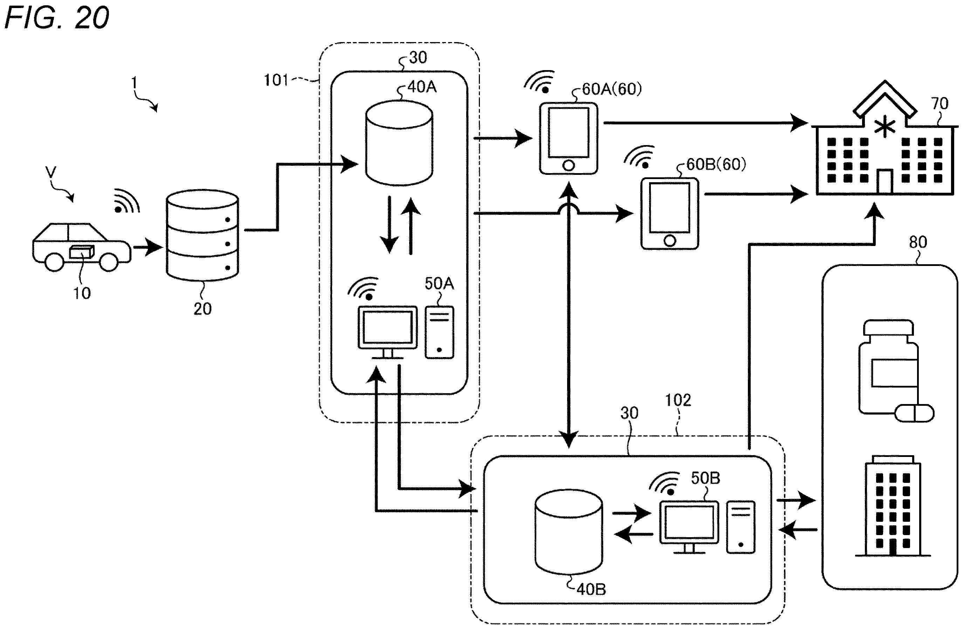

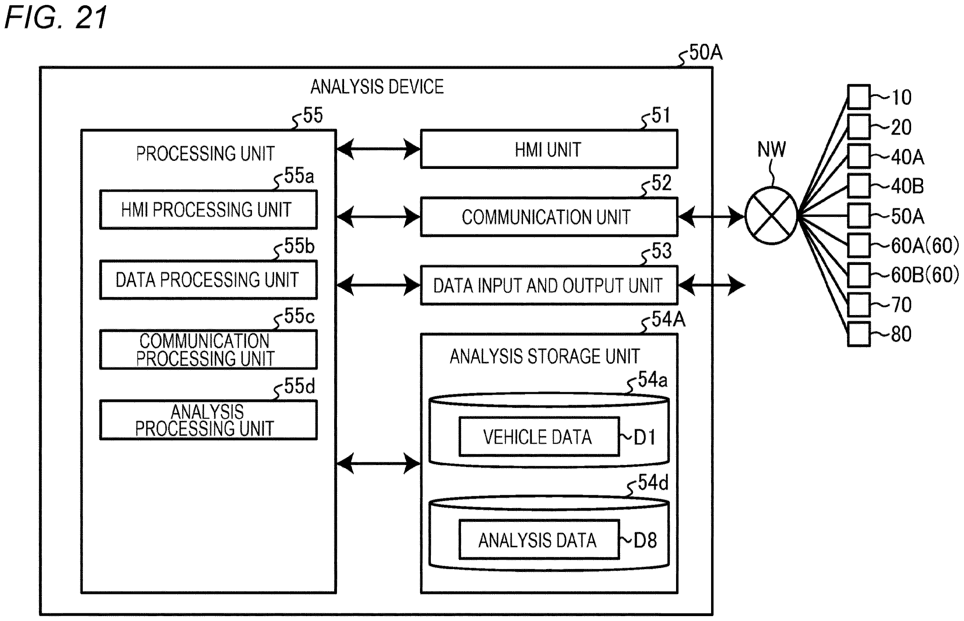

Next, a second embodiment will be described with reference to FIGS. 20 and 21. As shown in FIG. 20, the consultation recommendation system 1 and a base station device (the service providing device 30) according to the second embodiment are different from the consultation recommendation system 1 and the base station device (the service providing device 30) according to the first embodiment described above in that the vehicle data D1 is transmitted from the data processing device 20 to the medical server 40B via the mobility server 40A and the analysis device 50A. As shown in FIG. 21, the analysis storage unit 54A of the analysis device 50A also includes the vehicle data storage unit 54a in terms of functional concept. The vehicle data storage unit 54a is a storage area for storing the vehicle data D1. The analysis device 50A receives the vehicle data D1 transmitted from the mobility server 40A, and stores the vehicle data D1 in the vehicle data storage unit 54a. The analysis device 50A transmits the stored vehicle data D1 to the medical server 40B. Other configurations are substantially the same as those of the first embodiment.

### <Flow of Data in Consultation Recommendation System>

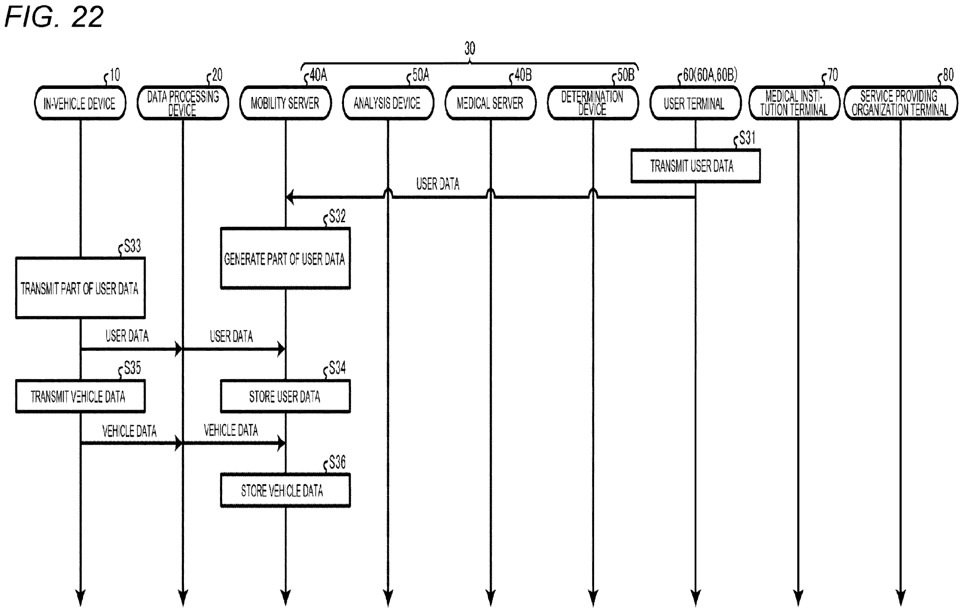

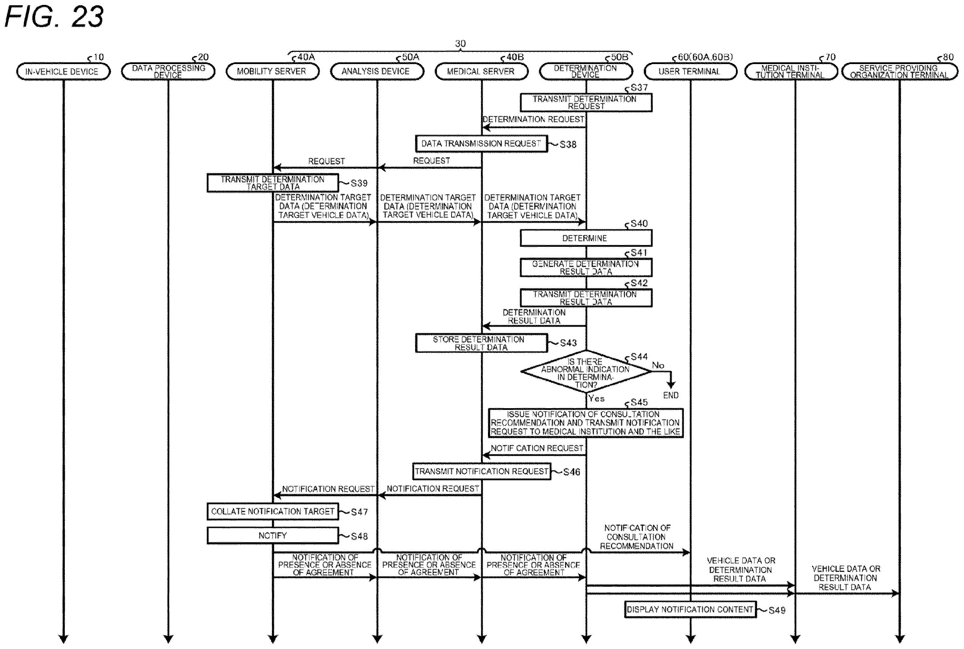

Next, an example of a flow of each process in the consultation recommendation system 1 according to the second embodiment will be described with reference to FIGS. 22 and 23. Similarly to the first embodiment, in the following description, transmission and reception of data are performed via the network NW, but the description thereof may be omitted. In the following description, in order to mainly focus on the flow of data, a description of specific processing operations related to the processing unit and the like in each device may be omitted as appropriate, and the outline of the entire configuration described above will be referred to as necessary. The flow of each process described below is merely an example of a representative process, and the present invention is not limited thereto.

### <Storage of User Data and Vehicle Data>

First, as shown in FIG. 22, the user terminal 60 (the first user terminal 60A) transmits, as the user data D2 to the mobility server 40A, data related to the user basic information D20, the service setting information D23, the favorite information D25, and the like registered in the user terminal 60 (the first user terminal 60A) in the user data D2 (step S31), for example. The user terminal 60 (the first user terminal 60A) is capable of transmitting not only the initially registered user data D2 but also the user data D2 updated after the initial registration to the mobility server 40A. The user terminal 60 (the first user terminal 60A) can transmit, for example, the user basic information D20 including the terminal registration information of the second user terminal 60B as the user data D2 such that the consultation recommendation notification for the user is also notified to the second user terminal 60B mainly used by the family of the user, the related person, or the like. In a case where the user newly registers a service related to the notification of the consultation recommendation, in a case where the registration information is changed for the service related to the notification of the consultation recommendation that has already been registered, or the like, it is possible to register the vehicle identification information D10 in the user data D2 by including the vehicle identification information D10 in the terminal registration information using the user terminal 60 (the first user terminal 60A). Accordingly, even when the vehicle data D1 is not stored in the mobility server 40A, the user data D2 and the determination result data D4 can be managed in association with each other based on the vehicle identification information D10.

In response to receiving the user data D2 from the user terminal 60 (first user terminal 60A), the mobility server 40A automatically generates a part of the user data D2 based on the received user data D2 (step S32). Here, the mobility server 40A automatically generates data related to the user attribute information D21, the agreement history information D22, the contract package information D24, and the like based on data related to the user basic information D20, the service setting information D23, the favorite information D25, and the like received from the user terminal 60 (the first user terminal 60A), for example.

The user data D2 may be registered, for example, via the in-vehicle device 10. In this case, the in-vehicle device 10 can register a part of the information of the user data D2 in accordance with the operation on the HMI unit 12 performed by the user, and also can transmit data related to the registered part of the information as the user data D2 to the data processing device 20, and transmit the data to the mobility server 40A via the data processing device 20 (step S33).

The mobility server 40A stores the received user data D2 and the automatically generated user data D2 in the server storage unit 43 (user data storage unit 43c) in association with the corresponding user ID D50 based on the ID data D5 and the like stored in the server storage unit 43 (ID data storage unit 43b) (step S34).

The in-vehicle device 10 transmits the vehicle data D1 detected and acquired by the detection and acquisition unit 11 to the data processing device 20 at an appropriate timing (step S35). In this case, the in-vehicle device 10 transmits the vehicle data D1 to the data processing device 20. In this case, the vehicle data D1 includes the vehicle identification information D10. In response to receiving the vehicle data D1 from the in-vehicle device 10, the data processing device 20 transmits the received vehicle data D1 to the mobility server 40A.

In response to receiving the vehicle data D1 from the data processing device 20, the mobility server 40A stores the vehicle data D1 in the server storage unit 43 (the vehicle data storage unit 43a) (step S36).

### <Determination of Abnormal Indication of User and Notification of Consultation Recommendation>

As shown in FIG. 23, in response to acquiring the vehicle data D1 used to determine an abnormal indication of the user from the mobility server 40A, the determination device 50B transmits a data transmission request to the medical server 40B (step S37). In response to receiving the data transmission request from the determination device 50B, the medical server 40B transmits the data transmission request to the mobility server 40A via the analysis device 50A (step S38). In response to receiving the data transmission request, the mobility server 40Areads the vehicle data D1 to be determined. The mobility server 40A transmits the read vehicle data D1 to be determined to the medical server 40B via the analysis device 50A. In response to receiving the vehicle data D1 to be determined, the medical server 40B transmits the vehicle data D1 to the determination device 50B (step S39). In response to receiving the vehicle data D1 to be determined, the determination device 50B determines the presence or absence of an abnormal indication of the user who drives the vehicle V from the vehicle data D1 to be determined (step S40). In this case, the determination processing unit 55e of the determination device 50B determines the presence or absence of an abnormal indication of the user from the vehicle data D1 based on the determination reference data D6. Here, the abnormal indication of the user includes an indication of deterioration in cognitive function of the user determined based on the determination reference data D6, an abnormality of a driving behavior of the user detected based on the determination reference data D6, and the like.

Thereafter, the determination device 50B generates a determination result as the determination result data D4 and stores the generated determination result in the determination storage unit 54B (step S41). In the determination device 50B, the determination result data D4 is managed in association with the vehicle identification information D10 included in the vehicle data D1 corresponding to the determination target. The determination device 50B transmits the determination result data D4 in association with the vehicle identification information D10 to the medical server 40B (step S42). In response to receiving the determination result data D4, the medical server 40B stores the determination result data D4 in association with the vehicle identification information D10 in the server storage unit 43 (the determination result data storage unit 43e) and manages the determination result data D4 (step S43). The determination device 50B confirms whether the determination result is a result that there is an abnormal indication in the user who drives the vehicle V (step S44). Here, when the determination result is a result that there is no abnormal indication in the user who drives the vehicle V, the determination device 50B ends the process until the next determination is performed. On the other hand, when the determination result is a result that there is an abnormal indication in the user who drives the vehicle V, the determination device 50B issues a notification of a consultation recommendation to the user to be determined (step S45). Here, the determination device 50B may issue a notification to a medical institution or a service providing organization together with the notification of the consultation recommendation. The notification of the consultation recommendation is issued by the determination device 50B transmitting a notification request to the medical server 40B. Here, the notification request requests the user corresponding to the vehicle identification information D10 associated with the determination result data D4 to be issued the notification of the consultation recommendation. The notification request includes the vehicle identification information D10 usable for specifying the user. When the notification request is made, the determination device 50B may transmit the determination result data D4 in step S42 simultaneously with the transmission of the notification request. A notification to the medical institution or the service providing organization is issued by the determination device 50B transmitting the vehicle data D1 or the determination result data D4 to the medical institution or the service providing organization. Here, when the vehicle data D1 or the determination result data D4 is to be transmitted to the medical institution or the service providing organization, the determination device 50B performs, on the mobility server 40A, agreement confirmation as to whether the user agrees to provide the vehicle data D1 or the determination result data D4 to the medical institution or the service providing organization. The agreement confirmation is included in the notification request from the determination device 50B to the medical server 40B.

In response to receiving the notification request from the determination device 50B, the medical server 40B transmits the notification request to the mobility server 40A via the analysis device 50A (step S46). In response to receiving the notification request, the mobility server 40A performs collation on a notification target of the consultation recommendation (step S47). In the collation of the notification target, the vehicle identification information D10 included in the vehicle data D1 and the user data D2 are stored in association with each other, and the vehicle identification information D10 included in the vehicle data D1 and the determination result data D4 are stored in association with each other, and thus the mobility server 40A specifies the user terminal 60 from the user data D2 corresponding to the determination result data D4 based on the vehicle identification information D10 included in the vehicle data D1 used in the determination by the determination device 50B, and performs collation on the notification target of the consultation recommendation. Specifically, the determination device 50B transmits the notification request including the vehicle identification information D10 corresponding to the notification target of the consultation recommendation to the mobility server 40A, and the mobility server 40A specifies the user data D2 corresponding to the notification request and the determination result data D4 by collating the vehicle identification information D10 included in the notification request, the vehicle identification information D10 associated with the determination result data D4, and the vehicle identification information D10 associated with the user data D2. The mobility server 40A specifies the user terminal 60 to which the notification of the consultation recommendation is transmitted, based on the terminal registration information of the user terminal 60 included in the service setting information D23 of the specified user data D2. The mobility server 40A performs agreement confirmation as to whether the user agrees to provide the vehicle data D1 or the determination result data D4 to the medical institution or the service providing organization. As described above, the mobility server 40A specifies the user of a notification target of the consultation recommendation from the service setting information D23 included in the user data D2 by collating the vehicle identification information D10 included in the notification request, the vehicle identification information D10 associated with the determination result data D4, and the vehicle identification information D10 associated with the user data D2. Further, the mobility server 40A can confirm whether the user agrees to provide the vehicle data D1 or the determination result data D4 to the medical institution or the service providing organization from the agreement history information D22 included in the user data D2.

Thereafter, the mobility server 40A issues, to the user, a notification of the consultation recommendation by transmitting information related to the consultation recommendation to the specified user terminal 60 (step S48). The mobility server 40A transmits, to the determination device 50B, information on whether the user agrees to provide the vehicle data D1 or the determination result data D4 to the medical institution or the service providing organization. In response to receiving the information indicating that the user does not agree, the determination device 50B transmits the vehicle data D1 or the determination result data D4 to the medical institution or the service providing organization. Here, when transmitting the determination result data D4, the determination device 50B transmits the determination result data D4 in association with the vehicle identification information D10 included in the vehicle data D1. When the determination result data D4 is transmitted in association with the vehicle identification information D10, for example, in a case where the user who has received the notification of the consultation recommendation makes a consultation reservation from the user terminal 60 (the first user terminal 60A), the medical institution can associate the acquired determination result data D4 with the user who has made the consultation reservation based on the vehicle identification information D10. With this configuration, the medical institution can more accurately grasp the state of the user. In this case, the consultation reservation made by the user terminal 60 (the first user terminal 60A) includes the vehicle identification information D10. By transmitting the determination result data D4 in association with the vehicle identification information D10, for example, the service providing organization can provide the user with information related to various services via the service providing device 30. In response to receiving the information indicating that the user does not agree, the determination device 50B does not transmit the vehicle data D1 or the determination result data D4 to the medical institution or the service providing organization.

In response to receiving the information related to the consultation recommendation from the mobility server 40A, the user terminal 60 displays a notification content of the consultation recommendation on the display unit (step S49). For example, the user terminal 60 displays the behavior recommended to the user as shown in FIGS. 12 to 14 on the display unit according to the content of the determination result data D4.

### <Access from Service Providing Organization Terminal>

As shown in FIG. 19, the service providing organization terminal 80 can make an access request to the determination device 50B in order to use the vehicle data D1 and the determination result data D4 stored in the determination device 50B. First, when making the access request to the determination device 50B, the service providing organization terminal 80 transmits the access request to the determination device 50B (step S21). In response to receiving the access request, the determination device 50B transmits, to the mobility server 40A, an agreement confirmation request for confirming whether the user agrees with the use of the vehicle data D1 or the determination result data D4 by the service providing organization (step S22). Here, the agreement confirmation request is made via the medical server 40B and the analysis device 50A. The mobility server 40A confirms whether each user agrees with the use of the vehicle data D1 or the determination result data D4 by the service providing organization. The mobility server 40A permits the access of the service providing organization terminal 80 to the vehicle data D1 or the determination result data D4 of a user who agrees with the use of the vehicle data D1 or the determination result data D4 by the service providing organization. The mobility server 40A rejects the access of the service providing organization terminal 80 to the vehicle data D1 or the determination result data D4 of a user who does not agree with the use of the vehicle data D1 or the determination result data D4 by the service providing organization.

### <Effects of Second Embodiment>

As described above, in the consultation recommendation system 1 according to the second embodiment, the vehicle data D1 is transmitted from the vehicle V to the mobility server 40A, and is further transmitted from the mobility server 40A to the medical server 40B. The medical server 40B acquires only vehicle data D1 used by the determination device 50B to determine the presence or absence of an abnormal indication of the user among the vehicle data D1 stored in the mobility server 40A.

In the consultation recommendation system 1 according to the present embodiment, the medical server 40B directly acquires the vehicle data D1 from the vehicle V via the mobility server 40A. In this way, secret retention of the vehicle data D1 can be improved by setting the specific mobility server 40A as an acquisition source of the vehicle data D1.

In the consultation recommendation system 1 according to the present embodiment, the determination result data D4, which is personal information having high confidentiality, is managed by the medical server 40B, and the user data D2 is managed by the mobility server 40A different from the medical server 40B, so that it is possible to smoothly utilize data used for mobility services while improving secret retention.

The consultation recommendation system 1 and the base station device according to each embodiment of the present invention are not limited to each embodiment described above, and various modifications can be made within the scope described in the claims.

### <Modification>

In the above description, the consultation recommendation system 1 issues a notification of a consultation recommendation only when it is determined that there is an abnormal indication in the user, but the present invention is not limited thereto. For example, even in a case where it is determined that there is no abnormal indication in the user as a determination result based on the reference, when the determination result is close to a level at which it is determined that there is an abnormal indication in the user, a notification may be issued to recommend a counselor or an operation consultation window. In addition, the notification of the consultation recommendation at the medical institution may include the content of recommending to make a consultation with the counselor or the operation consultation window.

In the above description, when the consultation recommendation system 1 issues the notification of the consultation recommendation to the user, the consultation recommendation system 1 specifies the user terminal 60 from the user data D2 corresponding to the determination result data D4 based on the vehicle identification information D10, but the present invention is not limited thereto. For example, when the consultation recommendation system 1 issues the notification of the consultation recommendation to the user, the consultation recommendation system 1 may specify the user terminal 60 from the user data D2 corresponding to the determination result data D4 based on an ID of the in-vehicle device 10, a manufacturing number of the in-vehicle device 10, or a registrant number of a service by the consultation recommendation system 1, instead of the vehicle identification information D10. In this case, the user terminal 60 can be specified by including the ID of the in-vehicle device 10, the manufacturing number of the in-vehicle device 10, or the registrant number of the service in the vehicle data D1 of the in-vehicle device 10.

In the above description, the consultation recommendation system 1 is configured such that the devices constituting the system transmit and receive data to and from each other through communication, but the present invention is not limited thereto. For example, a part or all of the data treated in the consultation recommendation system 1 may be written to or read from a recording medium via the data input and output units 14, 22, 42, 53, 63, 73, and 83 to transfer the data. Specifically, the vehicle data D1 collected by the in-vehicle device 10 may be written to a recording medium by the data input and output unit 14, read from the recording medium by the data input and output unit 22, and transferred to the data processing device 20. In the data processing device 20, the vehicle data D1 stored in the medical server 40B or the mobility server 40A by an operator may be written to a recording medium by the data input and output unit 22, and in the service providing device 30, the vehicle data D1 may be read from the recording medium by the data input and output unit 42 of the medical server 40B or the mobility server 40A, so that the vehicle data D1 is collectively stored in the medical server 40B or the mobility server 40A from the data processing device 20.

In the above description, the vehicle V may be a private vehicle or the like owned or used by the user, or may be a rental car, a shared car, or the like shared by unspecified utilizers. In a case where the vehicle V is a vehicle shared by unspecified utilizers, when the utilizer reserves or registers the use of the shared vehicle V, the consultation recommendation system 1 executes a process of associating the user ID D50 with the vehicle ID D51 of the shared vehicle V from the in-vehicle device 10 or the user terminal 60 to the mobility server 40A.

Each of the processing units 16, 24, 44, 55, 65, 75, and 85 described above may be implemented by a single processor, and the process related to each function may be executed, or may be implemented by a combination of a plurality of independent processors, and the process related to each function may be executed by each processor executing a program. The processes executed by the processing units 16, 24, 44, 55, 65, 75, and 85 may be integrated into a single processing circuit or may be distributed to a plurality of processing circuits.

Each of the mobility server 40A and the analysis device 50A described above may be implemented by a single processor, and the process related to each function may be executed, or may be implemented by a combination of a plurality of independent processors, and the process related to each function may be executed by each processor executing a program. The processes executed by the mobility server 40A and the analysis device 50A may be integrated into a single processing circuit or may be distributed to a plurality of processing circuits.

Each of the medical server 40B and the determination device 50B described above may be implemented by a single processor, and the process related to each function may be executed, or may be implemented by a combination of a plurality of independent processors, and the process related to each function may be executed by each processor executing a program. The processes executed by the medical server 40B and the determination device 50B may be integrated into a single processing circuit or may be distributed to the plurality of processing circuits.

In the above description, the configuration in which the mobility server 40A and the medical server 40B exchange information via the analysis device 50A has been described as an example, but the configuration is not limited thereto. For example, the mobility server 40A and the medical server 40B may exchange information without going through the analysis device 50A.

The consultation recommendation system and the base station device according to the present embodiment may be configured by appropriately combining the components of the embodiments and the modifications described above.

### REFERENCE SIGNS LIST

- 1: consultation recommendation system
- 10: in-vehicle device
- 20: data processing device
- 30: service providing device (base station device)
- 40A: mobility server
- 40B: medical server
- 50A: analysis device
- 50B: determination device
- 60: user terminal
- 60A: first user terminal
- 60B: second user terminal
- 70: medical institution terminal
- 80: service providing organization terminal
- NW: network
- V: vehicle

## Claims

1. A consultation recommendation system (1) comprising:
a mobility server (40A) configured to store user data (D2) related to a user of a vehicle;
a medical server (40B) configured to store vehicle data (D1) including traveling information of the vehicle;
a determination device configured to determine the presence or absence of an abnormal indication of the user from the vehicle data, store determination result data (D4) including a determination result in the medical server (40B), and issue a notification related to a cognitive function to the user based on the determination result data (D4); and
a user terminal (60, 60A, 60B) configured to receive the notification from the determination device, wherein
the mobility server (40A) and the medical server (40B) cooperate to manage the user data (D2), the vehicle data (D1), and the determination result data (D4) in association with each other, and
when the determination device determines that there is an abnormal indication in the user, the determination device specifies a user terminal (60, 60A, 60B) based on the user data (D2) corresponding to the determination result, and transmits, to the user terminal (60, 60A, 60B), a notification of a consultation recommendation on a disease related to the cognitive function.

2. The consultation recommendation system according to claim 1, wherein
the vehicle data includes vehicle identification information for identifying the vehicle,
the mobility server further manages the vehicle data and stores the user data and the vehicle identification information in association with each other,
the medical server stores the determination result data and the vehicle identification information in association with each other, and
the determination device specifies the user terminal from the user data corresponding to the determination result data based on the vehicle identification information and transmits the notification of the consultation recommendation to the user terminal.

3. The consultation recommendation system according to claim 1 or 2, wherein
the notification of the consultation recommendation includes a presentation of a behavior recommended to the user, and
the user terminal includes an output unit configured to present the behavior to the user and an input unit configured to receive an operation of performing the behavior

4. The consultation recommendation system according to claim 1 or 2, wherein
the traveling information includes information related to a driving operation of the vehicle, and
the determination device determines the presence or absence of an abnormal indication of the user based on determination reference data including first reference information based on a correlation relation between the driving operation of the vehicle and a cognitive function state of the user, or second reference information based on a determination as to whether the driving operation of the vehicle corresponds to a predetermined driving operation.

5. The consultation recommendation system according to claim 1 or 2, wherein
the medical server acquires only vehicle data used by the determination device to determine the presence or absence of an abnormal indication of the user among the vehicle data stored in the mobility server

6. The consultation recommendation system according to claim 1 or 2, further comprising:
a medical institution terminal installed at a medical institution, wherein
the determination device is configured to transmit at least one of the determination result data and the vehicle data to the medical institution terminal.

7. Abase station device (30) comprising:
a mobility server (40A) configured to store user data (D2) related to a user of a vehicle;
a medical server (40B) configured to store vehicle data (D1) including traveling information of the vehicle; and
a determination device configured to determine the presence or absence of an abnormal indication of the user from the vehicle data (D1), store determination result data (D4) including a determination result in the medical server (40B), and issue a notification related to a cognitive function to the user based on the determination result data (D4), wherein
the mobility server (40A) and the medical server (40B) cooperate to manage the user data (D2), the vehicle data (D1), and the determination result data (D4) in association with each other, and
when the determination device determines that there is an abnormal indication in the user, the determination device transmits, to a user corresponding to the determination result, a notification of a consultation recommendation on a disease related to the cognitive function.
